# EUROPEAN PATENT SPECIFICATION

(11) **EP 2 147 098 B1**
(45) Date of publication and mention of the grant of the patent: **17.07.2013**
(21) Application number: 08746618.1
(22) Date of filing: 23.04.2008
(51) Int. Cl.: C12N 9/96, C11D 3/38, C11D 3/386, C12P 21/06, C12N 9/54

(54) **USE OF PROTEIN HYDROLYSATES TO STABILIZE METALLOPROTEASE DETERGENT FORMULATIONS**
VERWENDUNG VON PROTEINHYDROLYSATEN ZUR STABILISIERUNG VON METALLOPROTEASE-DETERGENS-FORMULIERUNGEN
UTILISATION D'HYDROLYSATS DE PROTÉINE POUR STABILISER DES FORMULATIONS DÉTERSIVES DE MÉTALLOPROTÉASE

(30) Priority: 30.04.2007 US 914965 P
(43) Date of publication of application: 27.01.2010
(73) Proprietor: Danisco US Inc., Palo Alto, CA 94304 (US)
(72) Inventor: LEE, Sang-Kyu, Palo Alto, California 94304 (US); WINETZKY, Deborah, S., Palo Alto, California 94304 (US)
(74) Representative: Forrest, Graham Robert
(86) International application number: PCT/US2008/061230
(87) International publication number: WO 2008/134343

(56) References cited:
- EP-A- 0 544 359
- EP-A- 1 359 157
- WO-A-2007/044993
- US-A- 3 296 094
- US-A- 3 325 364
- US-A- 5 039 446
- KAMMOUN R ET AL: "Protein size distribution and inhibitory effect of wheat hydrolysates on Neutrase(R)." BIORESOURCE TECHNOLOGY, vol. 90, no. 3, December 2003 (2003-12), pages 249-254, XP002491648 ISSN: 0960-8524
- MUNEKIYO S M ET AL: "Presence of peptide inhibitors in rattlesnake venoms and their effects on endogenous metalloproteases" TOXICON, ELMSFORD, NY, US, vol. 45, no. 3, 1 March 2005 (2005-03-01), pages 255-263, XP004742410 ISSN: 0041-0101

## Description

### FIELD OF THE INVENTION

The invention relates to the use of protein hydrolysate inhibitors to stabilize metalloprotease containing detergent formulations under storage conditions.

### BACKGROUND OF THE INVENTION

Enzymes are key active ingredients in many detergent formulations. Because they are catalytic, enzymes can be highly effective ingredients in degrading stains. However, because they are biological products, enzymes can also be among the most costly ingredients. Consequently, maintaining high enzyme activity throughout the life of the detergent formulation is critical to the success of products based on such detergent formulations.

Enzymes used in such formulations include proteases, lipases, amylases, cellulases, mannosidases, as well as other enzymes or mixtures thereof.

Problems associated with maintaining enzyme stability during storage *(i.e.,* prior to use) in liquids are well known. Typically, proteases pose greater stability problems because their catalytic activity acts to degrade other proteins in the formulation, as well as the protease itself through the process of autolysis (*i.e.*, self degradation). However, due to the relative ease with which serine proteases (*e.g*., subtilisin) can be stabilized as well as the development of mutants engineered for increased stability, this class of protease have been used extensively in detergent formulations. Indeed, the subtilisins are among the most commercially important protease enzymes due to their use in detergents.

In contrast, metalloproteases have found little or no use in industrial applications such as detergent formulations. Metalloproteases involve more complex protein systems that have the absolute requirement for calcium and metal ions for stability and function, respectively. Detergent formulations and other cleaning compositions typically include a complex combination of active ingredients which greatly complicates the problem of maintaining metalloprotease stability and activity. In particular, detergent formulations often include compounds that chelate the calcium and/or essential metal ions resulting in a decrease in loss of stability and catalytic activity.

U.S. 2008/0293610 (11/581,102, corresponding to WO 2007/044993) discloses neutral metalloproteases which overcome some of the difficulties associated with the use of metalloproteases in detergent formulations. In particular, the neutral metalloproteases from *Bacillus sp.* NprE and PMN have been found to tolerate detergent formulation conditions and exhibit stability over approximately 4 weeks in the presence of zinc ion concentrations lower than 15 mM. Moreover, these neutral metalloproteases exhibit good cleaning effectiveness even at lower temperatures. In particular, the recombinant neutral metalloprotease from *Bacillus amyloliquefaciens,* NprE, has been shown to exhibit better wash performance on Equest Grass (Warwick) than other detergent formulations. Thus, neutral metalloproteases, if stabilized sufficiently, have potential for creating improved, commercially viable, industrial detergents.

Still, the neutral metalloproteases suffer from the common protease problem of autolytic degradation that rapidly decreases their activity and consequently, their storage stability of detergent formulations. The relative high cost of using metalloproteases (or any enzyme) requires minimizing any loss of activity prior to use to make them commercially viable as detergent ingredients. Obviously, any compositions and/or methods for minimizing autolytic degradation of metalloproteases must not further increase costs too greatly by inhibiting desired enzyme activity (*i.e*., degrading protein components of stains, etc.) during use. Consequently, a delicate balance must be struck in order to minimize autolytic activity during detergent storage without decreasing desired activity during use. Thus, there remains a need for methods, compounds, formulations, and compositions that stabilize neutral metalloproteases against degradation, particularly when they are incorporated in detergent formulations and other cleaning compositions.

### SUMMARY OF THE INVENTION

This invention provides detergent formulations as described in the claims comprising a metalloprotease enzyme and a metalloprotease inhibitor and which thereby exhibit increased stability against degradation. The invention also provides methods for the preparation of these inhibitor-stabilized detergent formulations.

The detergent formulations disclosed herein all comprise a metalloprotease, and the increased stability is due to the inclusion of a metalloprotease inhibitor which binds the enzyme and thereby prevents autolytic degradation of the enzyme. Importantly, these inhibitor-stabilized detergent formulations are prepared such that the inhibitor binds and effectively attenuates metalloprotease degradation during storage, but is subsequently released providing active enzyme upon dilution when the detergent is used. The invention discloses protein hydrolysates as particularly effective metalloprotease inhibitors for stabilizing against degradation. In a particularly preferred embodiment, the protein hydrolysate inhibitor is prepared by hydrolysis of a protein substrate by the metalloprotease enzyme itself. The resulting hydrolysis product may be isolated and is a particularly effective competitive inhibitor for the metalloprotease that generated it.

Thus, the metalloprotease inhibitors are protein hydrolysates. The protein hydrolysates of the invention comprise peptide fragments generated by the hydrolysis (either enzymatic or non-enzymatic) of a range of proteins (*e.g*., casein). The protein hydrolysates useful as inhibitors with the present invention include, but are not limited to: wheat gluten hydrolysate (*e.g*., HyPep 4601^{™}), soy protein acid hydrolysate (*e.g*., Amisoy), casein acid hydrolysate from bovine milk (*e.g*., Amicase), and enzymic hydrolysate from vegetable protein (*e.g*., Proteose peptone). In addition, the invention teaches the use of protein hydrolysate inhibitors made by hydrolysis/digestion with one or more metalloprotease enzyme, for example, the metalloprotease enzyme of interest itself.

In a preferred set of embodiments, the invention provides liquid detergent formulations comprising neutral metalloproteases isolated from a *Bacillus sp,* and in particular, the recombinant neutral metalloprotease from *Bacillus amyloliquifaciens,* NprE.

The invention comprises a liquid detergent formulation comprising: (a) from about 1% to about 75% of a surfactant by weight; (b) from about 10% to about 95 % of water by weight; (c) from about 0.01% to about 5% of a neutral metalloprotease by weight; and (d) an amount of neutral metalloprotease inhibitor such that the inhibitor binds to at least about 90% of the neutral metalloprotease molecules, *i.e*., binds at the active site or binds at a site other than the active site and prevents or inhibits catalytic interaction of substrate with the active site, prior to use, and wherein appropriate dilution of the detergent formulation results in the inhibitor dissociating from at least about 25% of the bound neutral metalloprotease molecules. Typically, appropriate dilution occurs when the liquid detergent formulation is added to a large volume of wash water that results in a 200, 400, 500, 600, or even 1000-fold dilution of the detergent formulation.

In one embodiment of this formulation, greater than or equal to 45%, 65%, 75%, 85%, or even 95% of the inhibitor bound neutral metalloprotease enzyme is released into its inhibitor-free form upon dilution of said detergent. In one embodiment, the inhibitor selected for the formulation competitively inhibits the neutral metalloprotease with an apparent Kᵢ of between about 5 mM to about 15 mM at between about pH 6.5 and about pH 11, preferably at between about pH 7.5 and about pH 9.5. In one preferred embodiment, the detergent formulation comprises a protein hydrolysate inhibitor with an apparent Kᵢ ~ 10 mM at about pH 8.0.

Although the absolute amount of inhibitor used in the formulation may vary depending on binding affinity, enzyme concentration and other factors, typically the amount of inhibitor is between about 0.01% and about 15%, about 0.05% and about 5%, about 0.1% and about 2.5%, by weight of the liquid detergent formulation before the appropriate dilution.

In one embodiment, the liquid detergent formulation is further stabilized by the presence of other ingredients including polypropylene glycol and/or calcium ions (*e.g.*, CaCl₂). In some embodiments, the formulation is an HDL detergent made according to a generic HDL formulation selected from the group consisting of: DW-AA, DW-AF, DW-AK, DW-CR, DW-CS, and DW-CT. In some embodiments, the liquid detergent formulation does not comprise boron.

A liquid detergent formulation of the invention may be made by a process of combining ingredients comprising: (a) an aqueous buffer at pH between about 6.5 and about 8.5; (b) from about 1% to about 75% of a detergent surfactant by weight; (c) from about 0.01% to about 5% of a metalloprotease by weight; and (d) a substrate protein, wherein digestion of the substrate protein by at least one, *i.e*., one or more, metalloprotease generates a product that binds to at least about 90% of the metalloprotease molecules. In one embodiment, the substrate protein is selected from the group consisting of: wheat gluten, casein, soy protein, and vegetable protein. In one embodiment, the substrate protein used is from about 0.01% to about 15% by weight. As with the other formulations disclosed herein, in some embodiments the metalloprotease is a neutral metalloprotease isolated from *Bacillus sp.,* and in particular, the neutral metalloprotease, NprE.

In another embodiment, the present invention provides a method for preparing an inhibitor-stabilized liquid detergent formulation comprising: (a) incubating a mixture comprising at least one, *i.e.,* one or more, neutral metalloprotease and a protein substrate in an aqueous buffer at pH between about pH 6.5 and about pH 11 and at a temperature from about 22°C to about 37°C, whereby digestion of the substrate protein by at least one, *i.e*., one or more, metalloprotease generates a hydrolysis product; (b) isolating the hydrolysis product with molecular weight less than about 5000 Da; and (c) combining the hydrolysis product of step (b) with a liquid detergent formulation comprising from about 0.001% to about 10% of a neutral metalloprotease by weight. In another embodiment, the invention provides a method for preparing an inhibitor-stabilized liquid detergent formulation comprising combining a protein hydrolysis product with a liquid detergent formulation comprising from about 0.001% to about 10% of a neutral metalloprotease by weight, wherein the protein hydrolysis product is prepared by incubating a mixture comprising at least one, *i.e*., one or more, neutral metalloprotease and a protein substrate in an aqueous buffer at pH between about pH 6.5 and about pH 11 and at a temperature from about 22°C to about 37°C, whereby digestion of the substrate protein by at least one, *i.e*., one or more, metalloprotease generates the hydrolysis product, and wherein hydrolysis product with molecular weight less than about 5000 Da is isolated prior to combining with the neutral metalloprotease. In one embodiment, the incubation mixture comprises from about 0.001% to about 10% neutral metalloprotease and from about 5% to about 20% protein substrate by weight. In one preferred embodiment, the neutral metalloprotease is NprE and the protein substrate is bovine milk casein.

### BRIEF DESCRIPTION OF THE DRAWINGS

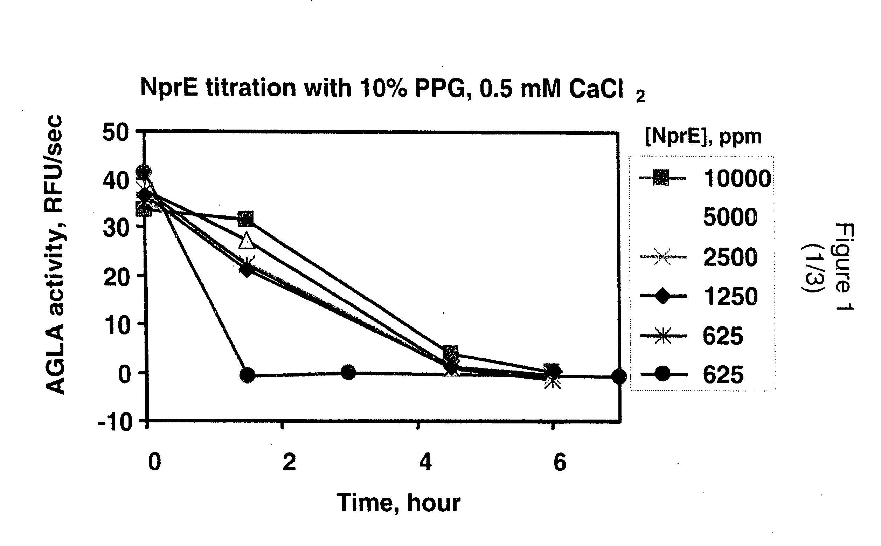

**Figure 1** depicts a plot of experimental assay data showing that NprE at higher concentrations with added polypropylene glycol (PPG) and CaCl2 retains increased AGLA activity over time. All samples contained the NprE concentration as listed in key along with 10% PPG and 0.5 mM CaCl₂. The closed circle data represents a control of 625 ppm NprE and without any added PPG or CaCl₂.

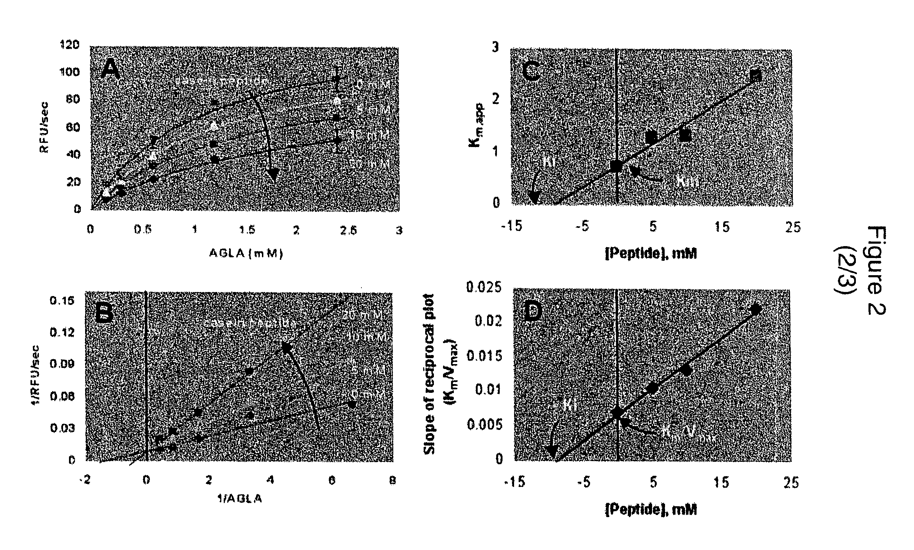

**Figure 2** depicts the steady-state kinetic data showing that an NprE-generated casein hydrolysis product is a competitive inhibitor of NprE. Figure 2A shows substrate dependence of NprE activity with the increasing amount of inhibitor. Figure 2B shows a double reciprocal plot exhibiting a common y-intercept. Figure 2C shows an apparent Kₘ replot against inhibitor peptide concentration. Figure 2D shows a double reciprocal slope replot against increasing amount of inhibitor peptide concentration. The x-intercept in Figures 2C and 2D indicates an apparent Kᵢ of approximately 10 mM casein hydrolysis product concentration.

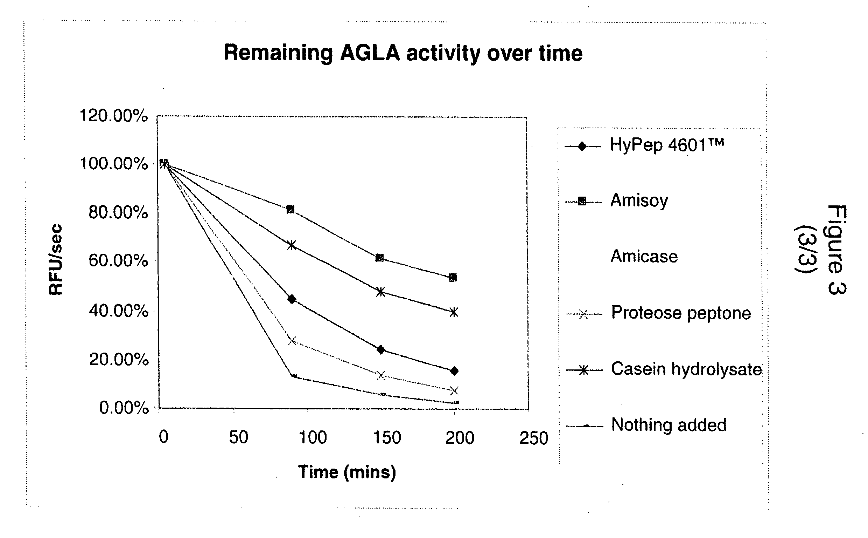

**Figure 3** depicts a plot of experimental assay data of NprE activity over time in the presence of various protein hydrolysates.

### DETAILED DESCRIPTION OF THE INVENTION

### Overview

This invention provides detergent formulations as described in the claims comprising a metalloprotease enzyme and a metalloprotease inhibitor which exhibit increased stability against degradation. The invention also provides methods for the preparation of these inhibitor-stabilized detergent formulations.

The detergent formulations disclosed herein all comprise a metalloprotease, and the increased stability is due to the inclusion of a competitive inhibitor which reversibly binds to the enzyme and thereby prevents autolytic degradation of the enzyme. The inhibitor as described herein may bind at the active site and directly interfere with interaction of substrate with the enzyme at the active site. Alternatively the inhibitor may bind at a site other than the active site (*i.e.,* non-specific to the active site) and prevent or inhibit catalytic interaction of substrate with the enzyme, for example, by induction of tertiary or quaternary structural alteration that interferes with or impedes substrate interaction at the active site. Importantly, these inhibitor-stabilized detergent formulations are prepared such that the inhibitor binds and effectively attenuates metalloprotease degradation during storage, but is subsequently released providing active enzyme upon dilution when the detergent is used. The invention discloses protein hydrolysates as particularly effective metalloprotease inhibitors for stabilizing against degradation. In particularly preferred embodiment, the protein hydrolysate inhibitor is prepared by hydrolysis of a protein substrate by the metalloprotease enzyme itself. The resulting hydrolysis product may be isolated and is a particularly effective competitive inhibitor for the metalloprotease that generated it.

### Definitions

Unless defined otherwise herein, all technical and scientific terms used herein have the same meaning as commonly understood by one of ordinary skill in the art to which this invention pertains. For example, Singleton and Sainsbury, Dictionary of Microbiology and Molecular Biology, 2d Ed., John Wiley and Sons, NY (1994); and Hale and Marham, The Harper Collins Dictionary of Biology, Harper Perennial, NY (1991) provide those of skill in the art with a general dictionaries of many of the terms used herein. Although any methods and materials similar or equivalent to those described herein find use in the practice of the present invention, the preferred methods and materials are described herein. Accordingly, the terms defined immediately below are more fully described by reference to the specification as a whole. Also, as used herein, the singular terms "a," "an," and "the" include the plural reference unless the context clearly indicates otherwise. Unless otherwise indicated, nucleic acids are written left to right in 5' to 3' orientation; amino acid sequences are written left to right in amino to carboxy orientation, respectively. It is to be understood that this invention is not limited to the particular methodology, protocols, and reagents described, as these may vary, depending upon the context in which they are used by those of skill in the art.

It is intended that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

The citation of any document is not to be construed as an admission that it is prior art with respect to the present invention.

As used herein, the term "enzyme" refers to any protein that catalyzes a chemical reaction. The catalytic function of an enzyme constitutes its "activity" or "enzymatic activity." An enzyme typically is classified according to the type of catalytic function it carries out, *e.g*., hydrolysis of peptide bonds.

As used herein, "effective amount of enzyme" refers to the quantity of enzyme necessary to achieve the enzymatic activity required in the specific application (*e.g*., personal care product, cleaning composition, etc.). Such effective amounts are readily ascertained by one of ordinary skill in the art and are based on many factors, such as the particular enzyme variant used, the detergent application, the specific formulation of the detergent, and the like.

As used herein, the terms "protease" or "proteinase" refer to any enzyme that catalyzes the hydrolysis of peptide bonds in a protein.

As used herein, the terms "metalloprotease," "metalloproteinase," or "metallopeptidase," refer to a protease that requires a bound metal ion to carry out its catalytic activity.

As used herein, the term "neutral metalloprotease" refers to a metalloprotease that is optimally active at neutral pH and requires zinc ions for catalytic activity. Typically, neutral metalloproteases are in the 30 to 40 kDa size range. The neutral metalloproteases of the present invention are also referred to as "neutral metalloendopeptidases" and include the enzymes in class EC 3.4.24.4.

As used herein, the term "substrate" refers to a substance (*e.g*., a chemical compound) on which an enzyme performs its catalytic activity to generate a product. In the case of metalloproteases, the substrate is usually a protein, although metalloproteases may also act on peptide or ester bonds in non-protein compounds. Thus, the term "protein substrate" refers to a substrate that is a protein.

As used herein, the term "active site" refers to the region of an enzyme at which the substrate binds and the catalytic activity occurs. In some cases, an enzyme may have more than one active site. Typically, metalloproteases have a single active site.

As used herein, the term "inhibitor" refers to any substance that reduces the rate of enzymatic activity. For example, inhibitor can refer to a protein hydrolysate, a polypeptide, or a natural or synthetic analog of a protein hydrolysate or polypeptide. Thus, inhibitors may include synthetic compounds that mimic certain aspects of a protein hydrolysate's ability to bind to a neutral metalloprotease enzyme's active site.

As used herein, the term "competitive inhibitor" refers to an inhibitor that binds reversibly to an enzyme and thereby prevents a substrate from binding, *i.e*., the inhibitor competes with the substrate for binding to the active site or the inhibitor binds elsewhere on the enzyme molecule and prevents or inhibits catalytic substrate interaction with the enzyme at the active site.

As used herein, "Kᵢ" or "inhibition constant" refers to the dissociation constant of an enzyme-inhibitor binding complex, *i.e*., the ratio of free enzyme concentration (*i.e*., "[E]") to inhibitor-bound enzyme (*i.e.,* "[E-I]"). Kᵢ can be determined using the well-known techniques of steady-state enzyme kinetics as described in most biochemistry textbooks *(see e.g*., Fersht, "Enzyme Structure and Mechanism," W.H. Freeman, 2nd Ed., 1985). Briefly, the inhibition constant, Kᵢ is determined by measuring the effect of an inhibitor's presence (*i.e*., inhibitor concentration) on the enzyme's steady-state kinetic constants (*i.e*., Kₘ and k_{cat}) in an assay with a known substrate.

As used herein, "apparent Kᵢ" refers to the value Kᵢ measured when the actual inhibitor concentration cannot be determined accurately. For example, when the inhibitor is a hydrolysis product mixture (*i.e.,* a mixture of protein fragments), the Kᵢ measured will be an "apparent Kᵢ" because the absolute inhibitor concentration can only be estimated, *e.g*., based on a chemical analysis of peptide concentration. The actual concentrations of the particular fragment(s) in the mixture that are binding to the enzyme molecule and resulting in the inhibition will be much lower than the measured concentration. Consequently, the "apparent Kᵢ" will be higher than then the Kᵢ value that would be determined using the purified inhibitor.

As used herein, the term "autolysis" refers to the lysis of a tissue or cell by its own enzymes. In one embodiment, the term "autolysis" refers to an enzyme's hydrolysis of its own polypeptide chain, *e.g*., self-proteolysis by a protease enzyme.

As used herein, the term "stability" in reference to an enzyme refers to its ability to maintain a certain level of functional activity over a period of time under certain environmental conditions. The term "stability" can be used in a number of contexts referring to the particular environmental condition that is of interest. For example, "autolytic stability" refers to the ability of an enzyme to withstand autolytic degradation (*i.e.*, self-proteolysis). A substantial change in stability is evidenced by at least about a 5% or greater increase or decrease (in most embodiments, it is preferably an increase) in the half-life of the enzymatic activity, as compared to the enzymatic activity present in the absence of the stabilizer (*e.g*., inhibitor compound). It is not intended that the term be limited to the use of any particular protease to assess the stability of a protein.

As used herein, the term "enzymatic conversion" refers to the modification of a substrate or intermediate to a product, by contacting the substrate or intermediate with an enzyme. In some embodiments, contact is made by directly exposing the substrate or intermediate to the appropriate enzyme. In other embodiments, contacting comprises exposing the substrate or intermediate to an organism that expresses and/or excretes the enzyme, and/or metabolizes the desired substrate and/or intermediate to the desired intermediate and/or end-product, respectively.

As used herein, the term "digestion" refers to the enzymatic conversion by a protease of a protein substrate into products.

As used herein, the terms "purified" and "isolated" refer to the removal of contaminants from a sample and/or removal of materials with which a substance (*e.g*., a polypeptide or polynucleotide) is naturally associated.

As used herein, the term "hydrolysate" refers to any substance produced by hydrolysis. The term is not intended to be limited to substance produced by any specific method of hydrolysis. The term is intended to include "hydrolysates" produced by enzymatic as well as non-enzymatic reactions. For example, any of the known hydrolytic enzymes (*e.g*., serine proteases, metalloproteases, hydrolases, etc.) are capable of producing hydrolysates within the meaning of how the term is used in the present application. Similarly, non-enzymatic methods of hydrolysis (*e.g*., acid/base hydrolysis, *etc.*) also produce hydrolysates within the meaning of how the term is used in the present application.

As used herein, the term "protein hydrolysate" refers to a hydrolysate produced by hydrolysis of a protein of any type or class. Any known protein may be hydrolyzed to produce a protein hydrolysate within the meaning of the term as used in the present application. A "protein hydrolysate" may be produced by enzymatic as well as non-enzymatic methods and may include protein fragments (*e.g*., polypeptides) that range in size from two to 100 or more amino acids. Further, as used herein, a "protein hydrolysate" is not limited to a single product compound, but may include a heterogenous distribution or mixture of hydrolysis products (*e.g*., protein fragments). It may also include a homogenous compound or purified fraction of hydrolysis products. Preferred embodiments of protein hydrolysates include: HyPep 4601™ (protein hydrolysate from wheat gluten), Amisoy (soy protein acid hydrolysate), Amicase (casein acid hydrolysate from bovine milk), Proteose peptone (enzymic hydrolysate from vegetable protein).

As used herein, "protein" refers to any composition comprised of amino acids and recognized as a protein by those of skill in the art. The terms "protein," "peptide" and "polypeptide" are used interchangeably herein. Wherein a peptide is a portion of a protein, those skilled in the art understand the use of the term in context. The terms "wild-type" and "native" are used to refer to proteins found in nature. In some embodiments, the wild-type protein's sequence is the starting point of a protein engineering project.

As used herein, the terms "related protein" or "homologous protein" refer to proteins that are functionally and/or structurally similar (*i.e*., have similar action and/or structure). It is intended that the term encompass the same or similar enzyme(s) (*i.e*., in terms of structure and function) obtained from different species. It is not intended that the present invention be limited to related proteins from any particular source(s) or proteins related evolutionarily. In addition, the terms related or homologous proteins encompass tertiary structural homologs and primary sequence homologs. Thus, the terms include proteins with "variant" or "mutant" sequences relative to the wild-type sequence.

As used herein, the terms "detergent," "detergent composition," and "detergent formulation" refer to mixtures which are intended for use in a wash medium for the cleaning of soiled objects. In some embodiments, the terms are used in reference to laundering fabrics and/or garments (*e.g*., "laundry detergents"). In other embodiments, the term refers to detergents such as those used to clean dishes, cutlery, etc. (*e.g*., "dishwashing detergents"). Generally, the terms are intended to encompass formulations, including "heavy duty liquid" ("HDL") formulations, comprising *e.g.*, metalloprotease enzymes, metalloprotease inhibitors such as protein hydrolysates, enzyme stabilizers such as polypropylene glycol, surfactants, transferase(s), hydrolytic enzymes, oxido reductases, builders, bleaching agents, bleach activators, bluing agents and fluorescent dyes, caking inhibitors, masking agents, enzyme activators, antioxidants, and solubilizers. It is not intended that the present invention be limited to any particular detergent formulation or composition.

As used herein, the term "surfactant" refers to a surface active compound that reduces surface tension. This term is intended to encompass all of the well-known types of surfactants and surfactant systems including nonionic surfactants, anionic surfactants, cationic surfactants, ampholytic surfactants, zwitterionic surfactants, semi-polar nonionic surfactants, and mixtures thereof.

As used herein, the phrase "detergent stability" refers to the ability of a detergent composition to maintain its ability to clean soiled objects in a wash medium under certain environmental conditions and for a certain time period. Detergent stability may be used to refer to the stability during its storage lifetime (*i.e*., pre-use), or stability during use in the wash medium. Detergent stability may vary with the type of cleaning test being used to measure stability. Furthermore, detergent stability may correspond closely with the stability of particular active ingredients in the detergent formulation when those particular ingredients contribute substantially with the cleaning test.

As used herein, "cleaning composition" and "cleaning formulation," unless otherwise indicated, refer to compositions that find use in the removal of undesired compounds from items to be cleaned, such as fabric, dishes, contact lenses, other solid substrates, hair (shampoos), skin (soaps and creams), teeth (mouthwashes, toothpastes) etc. The term encompasses any materials/compounds selected for the particular type of cleaning composition desired and the form of the product (*e.g*., liquid, gel, granule, or spray composition), as long as the composition is compatible with the metalloprotease and other enzyme(s) used in the composition. The specific selection of cleaning composition materials are readily made by one of ordinary skill upon considering the surface, item or fabric to be cleaned, and the desired form of the composition for the cleaning conditions during use.

The terms "cleaning composition" and "cleaning formulation" further refer to any composition that is suited for cleaning, bleaching, disinfecting, and/or sterilizing any object and/or surface. It is intended that the terns include, but are not limited to detergent compositions (*e.g.*, liquid and/or solid laundry detergents and fine fabric detergents; hard surface cleaning formulations, such as for glass, wood, ceramic and metal counter tops and windows; carpet cleaners; oven cleaners; fabric fresheners; fabric softeners; and textile and laundry pre-spotters, as well as dish detergents).

Furthermore, the term "cleaning composition" and "cleaning formulation" as used herein includes, unless otherwise indicated, granular or powder-form all-purpose or heavy-duty washing agents, especially cleaning detergents; liquid, gel or paste-form all-purpose washing agents, especially the so-called heavy-duty liquid (HDL) types; liquid fine-fabric detergents; hand dishwashing agents or light duty dishwashing agents, especially those of the high-foaming type; machine dishwashing agents, including the various tablet, granular, liquid and rinse-aid types for household and institutional use; liquid cleaning and disinfecting agents, including antibacterial hand-wash types, cleaning bars, mouthwashes, denture cleaners, car or carpet shampoos, bathroom cleaners; hair shampoos and hair-rinses; shower gels and foam baths and metal cleaners; as well as cleaning auxiliaries such as bleach additives and "stain-stick" or pre-treat types.

As used herein, "fabric" encompasses any textile material. Thus, it is intended that the term encompass garments, as well as fabrics, yarns, fibers, non-woven materials, natural materials, synthetic materials, and any other textile material.

The term "recombinant DNA molecule" as used herein refers to a DNA molecule that is comprised of segments of DNA joined together by means of molecular biological techniques.

The term "recombinant oligonucleotide" refers to an oligonucleotide created using molecular biological manipulations, including but not limited to, the ligation of two or more oligonucleotide sequences generated by restriction enzyme digestion of a polynucleotide sequence, the synthesis of oligonucleotides (*e.g.*, the synthesis of primers or oligonucleotides) and the like.

The term "regulatory element" as used herein refers to a genetic element that controls some aspect of the expression of nucleic acid sequences. For example, a promoter is a regulatory element which facilitates the initiation of transcription of an operably linked coding region. Additional regulatory elements include splicing signals, polyadenylation signals and termination signals.

The term "promoter/enhancer" denotes a segment of DNA which contains sequences capable of providing both promoter and enhancer functions (for example, the long terminal repeats of retroviruses contain both promoter and enhancer functions). The enhancer/promoter may be "endogenous" or "exogenous" or "heterologous." An endogenous enhancer/promoter is one which is naturally linked with a given gene in the genome. An exogenous (heterologous) enhancer/promoter is one which is placed in juxtaposition to a gene by means of genetic manipulation (*i.e*., molecular biological techniques).

As used herein, "expression vector" refers to a DNA construct containing a DNA sequence that is operably linked to a suitable control sequence capable of effecting the expression of the DNA in a suitable host. Such control sequences include a promoter to effect transcription, an optional operator sequence to control such transcription, a sequence encoding suitable mRNA ribosome binding sites and sequences which control termination of transcription and translation. The vector may be a plasmid, a phage particle, or simply a potential genomic insert. Once transformed into a suitable host, the vector may replicate and function independently of the host genome, or may, in some instances, integrate into the genome itself.

The terms "plasmid," "expression plasmid," and "vector" are used herein interchangeably with the plasmid is the most commonly used form of vector at present. However, the invention is intended to include such other forms of expression vectors that serve equivalent functions and which are, or become, known in the art.

The term "introduced" in the context of inserting a nucleic acid sequence into a cell, means transformation, transduction or transfection. Means of transformation include protoplast transformation, calcium chloride precipitation, electroporation, naked DNA and the like as known in the art. *(See,* Chang and Cohen, Mol. Gen. Genet., 168:111 - 115 [1979]; Smith et al., Appl. Env. Microbiol., 51:634 [1986]; and the review article by Ferrari et al., in Harwood, Bacillus Plenum Publishing Corporation, pp. 57-72 [1989]).

As used herein, "host cells" are generally prokaryotic or eukaryotic hosts which are transformed or transfected with vectors constructed using recombinant DNA techniques known in the art. Transformed host cells are capable of either replicating vectors encoding the protein variants or expressing the desired protein variant. In the case of vectors which encode the pre- or prepro-form of the protein variant, such variants, when expressed, are typically secreted from the host cell into the host cell medium.

### Neutral Metalloprotease Enzymes

Metalloproteases are a diverse class of proteases found in bacteria, fungi, as well as in higher organisms. A bound metal ion at the active site of the metalloprotease allows the catalytic activation of a water molecule. The water molecule then functions as a nucleophile to cleave the carbonyl group of the peptide bond. A wide variety of sequence and structure exists in the class, but the great majority of metalloproteases includes a zinc ion bound at the active site. In some metalloproteases, the zinc ion may be replaced by another metal ion such as cobalt or nickel without loss of activity. As currently understood, the catalytic mechanism of metalloproteases involves a non-covalent tetrahedral intermediate that forms via the attack of a zinc-bound water molecule on the carbonyl group of the bond being cleaved by the enzyme.

Neutral metalloproteases (*i.e*., neutral metalloendopeptidases, EC 3.4.24.4) belong to a protease class that has an absolute requirement for zinc ions for catalytic activity. These enzymes are optimally active at neutral pH and are in the 30 to 40 kDa size range. Neutral metalloproteases bind between two and four calcium ions that contribute to the structural stability of the protein. The neutral metalloprotease family includes the bacterial enzyme thermolysin, and other thermolysin-like proteases ("TLPs"), as well as carboxypeptidase A (a digestive enzyme), and the matrix metalloproteases that catalyze reactions involved in tissue remodeling and degradation.

Probably the best characterized neutral metalloproteases, in terms of function and stability, are thermolysin and the TLPs. Much research has focused on engineering *Bacillus subtilis* thermolysins to increase their thermal stability. (*See e.g.,* Vriend et al., In, Tweel et al. (eds), Stability and Stabilization of Enzymes, Elsevier, pp. 93-99 [1993].) Numerous efforts have been undertaken to increase the stability of TLPs by altering structural determinants, identified through molecular modeling, that may prevent local unfolding processes that enhance autolysis and denaturation at high temperatures. (*See e.g.,* van den Burg et al., in Hopsu-Havu et al., (eds), Proteolysis in Cell Functions Manipulating the Autolytic Pathway of a Bacillus Protease. Biomedical and Health Research Vol. 13, IOS Press [1997] p. 576.) It has been reported that calcium ions can help prevent neutral metalloprotease autolysis. The *B. stearothermophilus* neutral protease has been stabilized against autolysis and proteolytic degradation by addition of calcium (See, Dürrschmidt et al., FEBS J., 272:1523-1534 [2005]).

Compositions and methods to engineer neutral metalloproteases, including NprE, with improved characteristics are provided in U.S. 2008/0293610. Among other aspects, U.S. 2008/0293610 provides compositions and methods suitable for the engineering of neutral metalloproteases that are independent of calcium in order to maintain their structural stability. In other embodiments provided therein, engineering of a neutral metalloprotease prevents the local unfolding in a particular secondary structural element that may prevent proteolysis.

Among the stable neutral metalloproteases described in U.S. 2008/0293610 are the wild-type metalloprotease from *Bacillus amylo liquefaciens* (*e.g.,* purified MULTIFECT^{®} Neutral; "PMN") and the recombinant neutral metalloprotease (*e.g., Bacillus amyloliquefaciens* neutral metalloprotease cloned into *Bacillus subtilis*) referred to as NprE.

In addition to the neutral metalloprotease from *Bacillus amyloliquefaciens,* the present invention contemplates the use of related enzymes from other sources, particularly *Bacillus sp.,* including but not limited to metalloprotease homologs obtained from: *B. cereus, B. cereus* E33L, *B. caldolyticus, B. pumulis, B. megaterium, B, subtilis amylosacchariticus, Brevibacillus brevis, Paenibacillus polymyxa (Bacillus polymyxa), B. stearothermophilus, B. thuringiensis, B. subtilis* and *S*. *aureus,* as well as aureolysin, extracellular elastase, and neutral protease B.

The metalloproteases useful with the embodiments of the present invention may be purified by removal of contaminating proteins and other compounds within a solution or preparation that are not metalloprotease. In some embodiments, recombinant metalloprotease is expressed in bacterial or fungal host cells and these recombinant metalloproteases are purified by the removal of other host cell constituents; the percent of recombinant metalloprotease polypeptides is thereby increased in the sample. In particularly preferred embodiments, the metalloproteases used in accordance with the present invention are substantially purified to a level of at least about 99% of the protein component, as determined by SDS-PAGE or other standard methods known in the art. In alternative preferred embodiments, the metalloproteases used in the present invention comprise at least about 99% of the protease component of the compositions. In yet other alternative embodiments, the metalloprotease is present in a range of at least about 90-95% of the total protein and/or protease.

Functional characterization of wild-type and variant metalloprotease enzymes may be accomplished via any means suitable and is preferably based on the assessment of properties of interest. For example, pH and/or temperature, as well as detergent and /or oxidative stability is/are determined in some embodiments of the present invention. Indeed, it is contemplated that metalloprotease enzymes having various degrees of stability in one or more of these characteristics (proteolytic or autolytic stability, detergent stability, pH, temperature, and/or oxidative stability) may be used in the context of the present invention.

One approach to improving enzymes for detergent formulation stability is to alter the structure of the enzyme itself - *i.e.*, development of enzymes with variant amino acid sequences that exhibit increased activity, and/or specificity, under detergent formulation conditions. For example, a number of protease variants are disclosed in the art. *See e.g.,* EP 0 130 756, which corresponds to U.S. Reissue Pat. No. 34,606 (Genencor); EP 0 214 435 (Henkel); WO 87/04461 (Amgen); WO 87/05050 (Genex); EP 0 260 105 (Genencor); WO 88/08028 (Genex); WO 88/08033 (Amgen); WO 95/27049 (Solvay); WO 95/30011 (Procter & Gamble); WO 95/30010 (Procter & Gamble); WO 95/29979 (Procter & Gamble); U.S. Pat. No. 5,543,302 (Solvay); EP 0 251 446 (Genencor); WO 89/06279 (Novozymes A/S); WO 91/00345 (Novozymes A/S); EP 0 525 610 A1 (Solvay).

Variant enzymes can differ from a parent protein and one another by a small number of amino acid residues. The number of differing amino acid residues may be one or more, preferably 1, 2, 3, 4, 5, 10, 15, 20, 30, 40, 50, or more amino acid residues. In some preferred embodiments, the number of different amino acids between variants is between 1 and 10. In some particularly preferred embodiments, related proteins and particularly variant proteins comprise at least 35%, 40%, 45%, 50%, 55%, 60%, 65%, 70%, 75%, 80%, 85%, 90%, 95%, 97%, 98%, or 99% amino acid sequence identity.

Several methods are known in the art that are suitable for generating variants of the enzymes of the present invention, including but not limited to site-saturation mutagenesis, scanning mutagenesis, insertional mutagenesis, random mutagenesis, site-directed mutagenesis, and directed-evolution, as well as various other recombinatorial approaches.

A range of neutral metalloprotease variant sequences have been described in U.S. 2008/0293610. These variant enzymes can differ in functional characteristics from the wild-type enzyme to varying degrees. To the extent, however, that any such variant neutral metalloprotease has autolytic activity and also is competitively inhibited by a protein hydrolysate, it may be used in accordance with the formulations and methods disclosed herein. Thus, any of the inhibitor-stabilized metalloprotease embodiments described herein may be employed not only with wild-type neutral metalloprotease enzyme but also with a range of active mutants and other variants.

Site-directed mutagenesis may be used to engineer a neutral metalloprotease active site in which a particular protein hydrolysate will exhibit inhibitor binding characteristics that are more favorable for improved stability in a detergent formulation. Methods for generating neutral metalloprotease "site evaluation libraries" (SELs) of mutants are disclosed in U.S. 2008/0293610. In accordance with the present invention, these SELs can be used to generate a library of active site mutants of a neutral metalloprotease that can then be screened for improved protein hydrolysate (or other inhibitor) binding characteristics that improve the ability to use these inhibitors to stabilize against autolytic degradation in detergent formulations and cleaning compositions.

### Protein Hydrolysates as Inhibitors and Stabilizers

Neutral metalloproteases, such as NprE, when stored in solution can experience significant losses of activity over time. Much of this loss of enzyme activity is attributable to autolysis, *i.e*., a neutral metalloprotease molecule catalytically proteolyses other metalloprotease enzyme molecules or even itself. Autolysis can irreversibly damage the enzyme's folded structure such that its function and activity are greatly attenuated or fully destroyed. Typically, this autolytic loss of activity is exacerbated by higher temperatures often used in washing conditions to which detergent formulations are exposed. Consequently, this loss of enzyme activity results in a direct loss of detergent stability.

Ideally, in order to minimize autolysis each neutral metalloprotease molecule should be blocked from engaging in its catalytic activity until it is ready to use. An inhibitor of an enzyme can block its activity; however inhibitor binding to an enzyme's active site is often extremely tight and irreversible. For example, so-called "suicide inhibitors" chemically alter an enzyme's active site such that it is not possible to regain any catalytic activity.

Methods are known for improving storage stability of serine proteases in detergent formulations by adding inhibitors, such as the boron-based inhibitor compounds (*e.g*., boric acid and various boronic acids). These boron-based inhibitors are known to reversibly inhibit serine protease enzymes. For example, discussion of the inhibition of the serine protease subtilisin by boronic acid is provided in Molecular & Cellular Biochemistry 51, 1983, pp. 5-32. Yet these boron-based inhibitors do not strongly inhibit metalloproteases, which function by a different catalytic mechanism than the serine proteases. Additionally, some regulatory agencies have begun raising questions regarding the safety of boron-based compounds and are considering limiting their release into the environment.

For the purposes of the present invention, inhibitors of metalloproteases are desired that block the autolytic activity of the metalloprotease in a reversible fashion. The inhibitors should bind tightly but reversibly when the metalloprotease is stored, and then dissociate from the enzyme molecule thereby allowing the enzyme to regain its activity when its catalytic function is desired. Furthermore, this reversible inhibition must be compatible with the environmental conditions present when the enzyme is an ingredient in a detergent formulation or other cleaning composition.

It is well known that serine protease enzymes retain greater activity when stored at higher concentrations. It is believed that this is due to binding of the autolytic product to the serine protease active site when the enzyme is at higher concentration. In other words, the autolytic product dissociation rate is greatly reduced. In effect, higher overall enzyme concentrations cause the autolysis product to act as an inhibitor. When the concentration is lowered (by dilution) the autolysis product can readily dissociate and the autolysis reaction can resume.

As disclosed below in Example 1, the neutral metalloproteases exhibit increased stability (*i.e*., retain greater activity over time) when stored at higher concentration. As with the serine protease enzymes, this increased stability with concentration likely indicates that autolytic products of the metalloprotease enzymes are acting as inhibitors.

The observation that neutral metalloproteases, such as NprE, undergo product inhibition of autolysis at high concentrations suggests that other hydrolysis products can act as inhibitors to stabilize against autolysis. Thus, in one embodiment the present invention comprises a neutral metalloprotease containing formulation (or composition) comprising a neutral metalloprotease and a protein hydrolysate. In one embodiment, the protein hydrolysate is generated by the neutral metalloprotease itself. For example, as disclosed in Example 2 below, a bovine protein, such as milk casein, is treated with an active neutral metalloprotease, such as NprE, to enzymatically generate a protein hydrolysate mixture. Typically, this enzymatically generated protein hydrolysate is a heterogenous mixture of peptide products of various sizes, *e.g*., the peptides resulting from the NprE catalyzed digestion of casein. In one embodiment, this enzymatically generated protein hydrolysate composition may be used as an inhibitor as-is. In other embodiments, this mixture it may be further isolated and/or purified so as to generate a more concentrated and/or homogenous protein hydrolysate composition.

In one embodiment of the present invention, a neutral metalloprotease generated protein hydrolysate is run through a 5000 Da MW cut-off membrane to generate a low molecular weight mixture. This low MW mixture is then added to a formulation containing neutral metalloprotease to stabilize it against autolytic degradation during storage.

In accordance with the present invention, the metalloprotease inhibitor should be a competitive inhibitor. By using a competitive inhibitor, which binds reversibly, substantially less metalloprotease can be used in the detergent formulation or other cleaning composition. The factors to consider in selecting a competitive inhibitor for generation of inhibitor-stabilized metalloprotease are: (1) the metalloprotease inhibitor should be chosen with a Kᵢ, and/or the inhibitor should be added in a sufficient amount, such that at least about 90% of the enzyme molecules in the detergent formulation (or cleaning composition) are bound to the inhibitor during storage (*i.e*., prior to use); and (2) the inhibitor must also be selected such that during usage, when the detergent formulation (or cleaning composition) is diluted with water (or other appropriate liquid) from about 10-fold to about 10,000-fold, or from about 10-fold to about 100,000-fold, at least about 25%, 50%, 75%, 95%, or more of the bound inhibitor is released from the enzyme molecules.

In one embodiment, the competitive inhibitor is present in an amount such that it binds at least about 90% of the metalloprotease molecules prior to dilution, and upon dilution with water (or other appropriate liquid) from about 10-fold to about 10,000-fold, or from about 10-fold to about 100,000-fold, the inhibitor dissociates from at least about 25%, 50%, 75%, 95%, or more of the bound enzyme molecules and those molecules are released in catalytically active form.

In one preferred embodiment of the present invention, the metalloprotease inhibitor is a protein hydrolysate prepared by hydrolysis of a protein by the metalloprotease. In one embodiment, the metalloprotease is NprE, and the inhibitor is the hydrolysis product of bovine milk casein generated by NprE. In another embodiment, the metalloprotease is NprE and the inhibitor is a protein hydrolysate selected from the group consisting of: wheat gluten hydrolysate (*e.g*., HyPep 4601^{™}), soy protein acid hydrolysate (*e.g*., Amisoy), casein acid hydrolysate from bovine milk (*e.g*., Amicase), enzymic hydrolysate from vegetable protein (*e.g*., Proteose peptone), and any combination thereof.

Numerous other protein hydrolysate mixtures are commercially available. For example, the following protein hydrolysates are listed in the Sigma Chemical catalog: Albumin hydrolysate; Casein acid hydrolysate vitamin free; Casein Hydrolysate; Casein hydrolysate broth; Casein magnesium broth; Casein yeast magnesium agar; Casein yeast magnesium broth; Edamin® K; Gelatin hydrolysate enzymatic; Gluten Enzymatic Hydrolysate from corn; Hy-Case P; Hy-Case® M; Lactalbumin hydrolysate; Liver Hydrolysate; N-Z-Amine® B; N-Z-Amine® BT; N-Z-Amine® YTT; Peptone; Peptone from casein, acid digest; Peptone from lactalbumin, enzymatic digest, readily soluble; Peptone from meat, peptic digest; Peptone from milk solids; Peptone from salmon; Peptone Hy-Soy® T; Peptone N-Z-Soy® BL 4; Primatone; Protein Hydrolysate Amicase®; Protein Hydrolysate N-Z-Amine® AS; Proteose Peptone; Soy protein acid hydrolysate; Tryptone; Tiyptose; and Vegetable Hydrolysate No. 2.

All of these protein hydrolysate mixtures share the common feature of including a mixture of peptide fragments from the hydrolysis of a protein. Based on this common feature, one of ordinary skill would recognize that each protein hydrolysate mixture represents a potential inhibitor of a metalloprotease. In accordance with the teachings of the present invention, one of skill could screen these protein hydrolysates for their ability to inhibit (and stabilize against autolysis) a desired metalloprotease. Indeed, many of these protein hydrolysate mixtures are based on the hydrolysis of a common protein (*e.g*., casein). Consequently, the mixture may reasonably be expected to include polypeptide fragments of similar structure and consequently, capable of similar function as an inhibitor of a metalloprotease. As explained further below, the function of the protein hydrolysate is easily determined using the well-known techniques of enzyme kinetics.

In one embodiment, the metalloprotease inhibitors useful with the present invention are competitive inhibitors selected based on their observed Kᵢ value with the metalloprotease of interest. Thus, in one embodiment, the apparent Kᵢ of the protein hydrolysates of the present invention can be measured using standard, well-known, steady-state enzyme kinetic techniques. In the case of the NprE generated casein hydrolysis products with MW less than about 5000 Da, the steady state kinetic analysis yields an apparent Kᵢ of about 10 mM.

As shown in Example 2 below, the neutral metalloprotease generated casein protein hydrolysis product composition acts as a competitive inhibitor of the enzyme. In one embodiment, the present invention contemplates the use of protein hydrolysates that exhibit an apparent Kᵢ of less than about 15 mM, about 10 mM, about 5 mM, about 0.5 mM, or less. Apparent Kᵢ values are determined because the nature of the enzyme generated hydrolysate compositions is that they are a heterogenous mixture of peptides, and some of the peptides likely are acting as weak inhibitors or not inhibiting the enzyme at all.

In the case of a relatively purified, relatively homogenous, protein hydrolysate composition, the measured Kᵢ would be expected to be approximately 100-fold to 1000-fold lower, in the range of Kᵢ ~ 1-10 µM.

Generally, the present invention contemplates that optimal inhibition occurs when the metalloprotease is in the presence of an inhibitor concentration that is about 5-fold to about 10-fold, about 5-fold to about 100-fold, or more times the measured value of Kᵢ for the inhibitor with the metalloprotease.

An inhibitor-stabilized metalloprotease composition could be used as a precursor for the preparation of liquid detergent formulations, or in other applications involving cleaning compositions.
A suitable inhibitor-stabilized metalloprotease composition may comprise from about 0.001% to about 10% by weight of a neutral metalloprotease, wherein a competitive inhibitor is bound to at least about 90% of said neutral metalloprotease molecules. This inhibitor-stabilized composition can be a liquid or dry (*e.g*., granular) preparation, or an encapsulated particle.

The preparation of a dry composition involves first preparing the inhibitor bound enzyme by contacting the enzyme in solution with a protein substrate (*e.g*., casein) or with a protein hydrolysate (*e.g*., Amisoy) at a concentration that would result in a population with at least about 90% of the metalloprotease molecules bound to hydrolysate inhibitor. This solution is then dehydrated, *e.g*., by lyophilization, freeze-drying, and/or other techniques well-known in the art of protein formulation. This resulting dried inhibitor-bound enzyme composition then optionally is stored for later use in the preparation of an inhibitor-stabilized metalloprotease liquid detergent formulation by reconstitution with water and other detergent formulation ingredients.

Alternatively, the inhibitor-stabilized metalloprotease composition could be used to prepare an encapsulated particle formulation.

Thus, the inhibitor-stabilized metalloprotease composition may be used to prepare a detergent formulation by the process of combining the composition with: (a) water; (b) from about 0.1% to about 75% of a detergent surfactant by weight; (c) from about 5% to about 15% propylene glycol by weight; and (d) from about 0.5 mM to about 5.0 mM Ca²⁺ ion.

The absolute amount of inhibitor used in the formulations of the present invention may vary depending on inhibitor binding affinity (*i.e*., Kᵢ), inhibitor molecular weight, enzyme concentration and other factors. Typically, however, the amount of inhibitor used in the liquid detergent formulation embodiments of the present invention is between about 0.01% and about 15%, about 0.05% and about 5%, or about 0.1% and about 2.5%, by weight of the before any dilution related to the use of the formulation for washing. A particular protein substrate may be engineered (*e.g*., using site-directed mutagenesis) such that it provides a hydrolysis product with more favorable inhibition characteristics once it undergoes enzymatic conversion by the specific neutral metalloprotease. This variant protein substrate could be used to prepare an inhibitor-stabilized neutral metalloprotease composition.

### Vectors and Host Cells Co-Expressing Protein Substrates to Stabilize Neutral Metalloprotease

An expression vector may be provided comprising a neutral metalloprotease gene and a protein substrate gene, wherein the protein substrate gene product is enzymatically converted by the expressed neutral metalloprotease to produce a protein hydrolysate inhibitor. The cloned vector comprising the neutral metalloprotease and the protein substrate genes can then be introduced into a host cell (via well-known techniques in cell transformation or transfection) and used to co-express the two protein gene products.

Because the protein substrate is co-expressed with the neutral metalloprotease, the protein substrate can immediately undergo enzymatic conversion by the metalloprotease thereby generating a protein hydrolysis product (*i.e*., a protein hydrolysate). As described herein, the resulting protein hydrolysate product is then capable of inhibiting the neutral metalloprotease, resulting in greater protection against autolytic degradation for the newly expressed enzyme.
The co-expression vector comprises necessary elements for efficient gene expression (*e.g*., a promoter operably linked to the gene of interest). In some embodiments, these necessary elements are supplied as the gene's own homologous promoter if it is recognized, (*i.e*., transcribed, by the host), a transcription terminator (a polyadenylation region for eukaryotic host cells) which is exogenous or is supplied by the endogenous terminator region of the neutral metalloprotease gene. If desired, a selection gene such as an antibiotic resistance gene that enables continuous cultural maintenance of plasmid-infected host cells by growth in antimicrobial-containing media is also included.
The genetic element controlling production of the protein substrate may be operably linked to a separate promoter such that the separate promoter only enhances production of the protein substrate but not the neutral metalloprotease protein. Consequently, expression of the vector in a suitable host results in the production of a greater amount of protein substrate gene product than the metalloprotease. This increased ratio of protein substrate to metalloprotease in the fermentation broth results in an increase in the amount of the hydrolysis product thereby enhancing its ability to bind to the metalloprotease molecule and inhibit autolytic degradation.
The genetic elements controlling production of the neutral metalloprotease and the protein substrate may be on separate vectors (*e.g*., plasmids) that are transformed in a single host. In this case, it is also preferred that the protein substrate gene is operably linked to a promoter that allows it to be produced in greater amounts than the metalloprotease.
The host cell and vector are typically selected such that the co-expressed proteins are secreted into the extracellular fermentation broth. The co-expression vector may be a plasmid that replicates in the host cell. Typically, the plasmid used includes the well-known elements necessary for plasmid replication. Alternatively, the plasmid may be designed to integrate into the host chromosome.

Techniques for recombinant cloning, expression, and fermentation of the neutral metalloprotease from *B. amyloliquifaciens* NprE, into a plasmid vector introduced in a *B*. *subtilis* host is disclosed in U.S. 2008/0293610. This NprE expression system may be adapted for co-expression of a protein substrate for the NprE enzyme, such as casein.

### Detergent Formulations and Cleaning Compositions

Inhibitor-stabilized metalloprotease compositions are useful in formulating various detergent formulations and cleaning compositions. These formulations and compositions may be advantageously employed for example, in laundry applications, hard surface cleaning, automatic dishwashing applications, as well as cosmetic applications such as dentures, teeth, hair and skin. However, due to the increased effectiveness in lower temperature solutions and the superior color-safety profile of the neutral metalloprotease enzymes of the present invention, the inhibitor-stabilized compositions are ideally suited for laundry applications.

In addition to those disclosed herein, a broad range of detergent formulations and cleaning compositions suitable for use with the inhibitor-stabilized metalloproteases described here are disclosed in U.S. 2008/0293610.

Unless otherwise noted, all component or composition levels provided herein are made in reference to the active level of that component or composition, and are exclusive of impurities, for example, residual solvents or by-products, which may be present in commercially available sources. Enzyme components' weights are based on total active protein. All percentages and ratios are calculated by weight unless otherwise indicated. All percentages and ratios are calculated based on the total composition unless otherwise indicated.

In the exemplified detergent formulations and cleaning compositions, the enzyme levels are expressed as pure enzyme by weight of the total composition and unless otherwise specified, the detergent ingredients are expressed by weight of the total compositions
The detergent formulations and cleaning compositions of the present invention comprise at least: (1) a surfactant, preferably a non-ionic or anionic surfactant; and (2) from about 10% to about 95% water on a weight basis; and (3) metalloprotease enzyme; and (4) a metalloprotease inhibitor.

In other embodiments, this simple detergent formulation may further comprise a variety of additional substances (*i.e.,* adjunct materials) selected from the group consisting of: additional surfactants, builders, chelating agents, dye transfer inhibiting agents, deposition aids, dispersants, additional enzymes, enzyme stabilizers, catalytic materials, bleach activators, bleach boosters, hydrogen peroxide, sources of hydrogen peroxide, preformed peracids, polymeric dispersing agents, clay soil removal/anti-redeposition agents, brighteners, suds suppressors, dyes, perfumes, structure elasticizing agents, fabric softeners, carriers, hydrotropes, processing aids and/or pigments.

In one embodiment, the invention provides a liquid detergent formulation comprising: (a) from about 1% to about 75% of a surfactant by weight; (b) from about 10% to about 95% of water by weight; (c) from about 0.01% to about 5% of a neutral metalloprotease by weight; and (d) an amount of neutral metalloprotease inhibitor such that the inhibitor binds to at least about 90% of the neutral metalloprotease molecules prior to use, and wherein appropriate dilution of the detergent formulation results in the inhibitor dissociating from at least about 25% of the bound neutral metalloprotease molecules. Typically, appropriate dilution occurs when the liquid detergent formulation is added to a large volume of wash water that results in a 200, 400, 500, 600, or even 1000-fold dilution of the detergent formulation.

In another embodiment of this liquid detergent formulation, greater than or equal to 45%, 65%, 75%, 85%, or even 95% of the inhibitor bound neutral metalloprotease enzyme is released into its inhibitor-free form upon dilution of said detergent. In one embodiment, the inhibitor selected for the formulation competitively inhibits the neutral metalloprotease with an apparent Kᵢ of between about 5 mM to about 15 mM at between about pH 6.5 and about pH 11, and preferably between about pH 7.5 and about pH 9.5. In one preferred embodiment, the liquid detergent formulation comprises a protein hydrolysate inhibitor with an apparent Kᵢ ~10 mM at about pH 8.0.
The metalloprotease inhibitor is a protein hydrolysate. In a preferred embodiment, the metalloprotease inhibitor is a protein hydrolysate prepared by hydrolysis of a protein by the metalloprotease. In another embodiment, the metalloprotease is NprE, and the inhibitor is the hydrolysis product of bovine milk casein generated by NprE. In another embodiment, the inhibitor is a protein hydrolysate selected from the group consisting of: wheat gluten hydrolysate (*e.g*., HyPep 4601^{™}), soy protein acid hydrolysate (*e.g*., Amisoy), casein acid hydrolysate from bovine milk (*e.g*., Amicase), enzymic hydrolysate from vegetable protein (*e.g*., Proteose peptone), and any combination thereof.

The neutral metalloprotease formulations stabilized with protein hydrolysate inhibitors of the present invention are particularly well-suited for use in heavy duty liquid (HDL) detergent formulations.

In one embodiment, the inhibitor-stabilized metalloprotease compositions of the present invention may be incorporated in a HDL detergent formulation, wherein the HDL detergent formulation comprises: between about 30% and 60% by weight water; between about 45% and 15% by weight actives, respectively; and wherein the ratio of HDL detergent formulation to inhibitor-stabilized metalloprotease composition is about 9 to 1 by volume.

In some embodiments, the HDL formulation comprises between about 33% and 53%, between about 35% and 51%, or between about 36% and 44% water by weight. In some embodiments, the HDL formulation comprises as low as about 40%, 38%, 36%, 34%, 32%, 30%, or lower % water by weight.

In one embodiment, the HDL detergent formulations of the present invention further comprise between about 5% and 15%, between about 7.5% and 12.5%, or at least about 10% polypropylene glycol.

In one embodiment, the HDL detergent formulations of the present invention comprise between about 20% and about 50% surfactants by weight. In some embodiments, the HDL formulation comprises a mixture of surfactants selected from the group consisting of: C12 ethoxylates (Alfonic 1012-6, Hetoxol LA7, Hetoxol LA4), sodium alkyl benzene sulfonates (*e.g*., Nacconol 90G), sodium laureth sulfate (*e.g*., Steol CS-370), and any combination thereof. In some embodiments, the HDL formulation comprises one or more surfactants selected from alkylbenzene sulfonates, alkylether sulfates, and alcohol ethoxylates.

In one embodiment, the HDL detergent formulation of the present invention comprises from about 35% to about 52% water by weight, and about 24% to about 40% surfactants by weight, wherein the surfactants comprise: Nacconol 90G, Alfonic 1012-6, and Steol CS-370. In another embodiment, the specific ratio by volume of water and surfactants in the HDL formulation (assuming a total of 90 parts) is about: 30 parts water, 17 parts Nacconol 90G, 13 parts Alfonic 1012-6, and 10 parts Steol CS-370. One of ordinary skill would immediately recognize that alternative HDL formulations useful with the present invention may be prepared using equivalent surfactants in similar amounts.

Listed in Tables 1-5 (below) are the recipes for a range of exemplary generic HDL formulations that may be used with the present invention. These generic formulations have varying amounts of water and other active ingredients and are formulated for a ratio of 90 parts detergent to 10 parts inhibitor-stabilized metalloprotease composition. In one preferred embodiment, the liquid detergent formulations comprise an inhibitor-stabilized metalloprotease (preferably a protein hydrolysate stabilized metalloprotease) and the ingredients of any one of the generic HDL detergent formulations in the same ratio as the recipes listed in Tables 1-5.

**Table 1: Generic HDL detergent formulations: DW-CR, DW-CS, and DW-CT.**

| **Ingredient** | **Formulation** | | |
|---|---|---|---|
| | **DW-CR** | **DW-CS** | **DW-CT** |
| Water, DI | 46.4 | 37.45 | 30.4 |
| Borax | 1.6 | 1.6 | 1.6 |
| Boric acid | 1 | 1 | 1 |
| Ethanol, 70% | 7 | 7 | 7 |
| Propylene glycol | 10 | 10 | 10 |
| Nacconol 90G | 10 | 13.3 | 16.67 |
| Alfonic 1012-6 | 8 | 10.7 | 13.33 |
| Steol CS370 | 6 | 8 | 10 |
| | | | |
| Total parts | 90.0 | 90.0 | 90.0 |
| Surfactant (wt %)¹ | 24 | 32 | 40 |
| Water (wt %)² | 51.192 | 43.21 | 36.392 |

| | | | |
|---|---|---|---|
| ¹ Includes Hetoxol LA7, Hetoxol LA4, Nacconol 90G and Steol CS370, all considered as 100% active. ² Includes water added as such in addition to the water present in borax, boric acid, ethanol and Steol CS370. Water from borax and boric acid is calculated from Na₂B₄O₅(OH)₄˙8H₂O. | | | |

**Table 2: Generic HDL detergent formulations: DW-AA, DW-AF, and DW-AK.**

| **Ingredient** | **Formulation** | | |
|---|---|---|---|
| | **DW-AA** | **DW-AF** | **DW-AK** |
| Water, DI | 46.4 | 38.4 | 30.4 |
| Borax | 1.6 | 1.6 | 1.6 |
| Boric acid | 1.0 | 1.0 | 1.0 |
| Propylene glycol | 10.0 | 10.0 | 10.0 |
| Ethanol, 70% | 7.0 | 7.0 | 7.0 |
| Hetoxol LA7 | 6.72 | 9.0 | 11.2 |
| Hetoxol LA4 | 1.28 | 1.7 | 2.13 |
| Nacconol 90G | 10.0 | 13.3 | 16.67 |
| Steol CS370 | 6.0 | 8.0 | 10.0 |
| | | | |
| Total parts | 90.0 | 90.0 | 90.0 |
| Surfactant (wt %)¹ | 24.0 | 32.0 | 40.0 |
| Water (wt %)² | 51.192 | 43.792 | 36.392 |
| pH (neat) | 8.1 | 8.0 | 8.0 |

| | | | |
|---|---|---|---|
| ¹ Includes Hetoxol LA7, Hetoxol LA4, Nacconol 90G and Steol CS370, all considered as 100% active. ² Includes water added as such in addition to the water present in borax, boric acid, ethanol and Steol CS370. Water from borax and boric acid is calculated from Na₂B₄O₅(OH)₄˙8H₂O. | | | |

**Table 3: Generic HDL detergent formulations: DW-CO, DW-CP, and DW-CQ.**

| **Ingredient** | **Formulation** | | |
|---|---|---|---|
| | **DW-CO** | **DW-CP** | **DW-CQ** |
| Water, DI | 47.272 parts | 39.272 parts | 31.272 parts |
| Phosphoric acid, 75% | 0.316 | 0.316 | 0.316 |
| TSP³ | 1.412 | 1.412 | 1.412 |
| Propylene glycol | 10.0 | 10.0 | 10.0 |
| Ethanol, 70% | 7.0 | 7.0 | 7.0 |
| Hetoxol LA7 | 6.72 | 9.0 | 11.2 |
| Hetoxol LA4 | 1.28 | 1.7 | 2.13 |
| Nacconol 90G | 10.0 | 13.3 | 16.67 |
| Steol CS370 | 6.0 | 8.0 | 10.0 |
| | | | |
| Total | 90.0 parts | 90.0 parts | 90.0 parts |
| Surfactant (wt %)¹ | 24.0 | 32.0 | 40.0 |
| Water (wt %)² | 51.172 | 43.772 | 36.372 |
| pH (neat) | 8.0 | 8.0 | 8.0 |

| | | | |
|---|---|---|---|
| ¹ Includes Hetoxol LA7, Hetoxol LA4, Nacconol 90G and Steol CS370, all considered as 100% active. ² Includes water added as such in addition to the water present in borax, boric acid, ethanol and Steol CS370. Water from borax and boric acid is calculated from Na₂B₄O₅(OH)₄˙8H₂O. ³ "TSP" = trisodium orthophosphate dodecahydrate, 0.25 M sodium hydroxide. | | | |

**Table 4: Generic HDL detergent formulations: DW-BL, DW-BN, and DW-BP.**

| **Ingredient** | **Formulation** | | |
|---|---|---|---|
| | **DW-BL** | **DW-BN** | **DW-BP** |
| Water, DI | 47.272 parts | 39.272 parts | 31.272 parts |
| Borax | 1.6 | 1.6 | 1.6 |
| Boric acid | 1.0 | 1.0 | 1.0 |
| Phosphoric acid, 75% | 0.316 | 0.316 | 0.316 |
| TSP³ | 1.412 | 1.412 | 1.412 |
| Propylene glycol | 10.0 | 10.0 | 10.0 |
| Ethanol, 70% | 7.0 | 7.0 | 7.0 |
| Hetoxol LA7 | 6.72 | 9.0 | 11.2 |
| Hetoxol LA4 | 1.28 | 1.7 | 2.13 |
| Nacconol 90G | 10.0 | 13.3 | 16.67 |
| Steol CS370 | 6.0 | 8.0 | 10.0 |
| | | | |
| Total | 90.0 parts | 90.0 parts | 90.0 parts |
| Surfactant (wt %)¹ | 24.0 | 32.0 | 40.0 |
| Water (wt %)² | 49.464 | 42.064 | 34.664 |
| pH (neat) | 8.0 | 8.0 | 8.0 |

| | | | |
|---|---|---|---|
| ¹ Includes Hetoxol LA7, Hetoxol LA4, Nacconol 90G and Steol CS370, all considered as 100% active. ² Includes water added as such in addition to the water present in borax, boric acid, ethanol and Steol CS370. Water from borax and boric acid is calculated from Na₂B₄O₅(OH)₄˙8H₂O. ³ "TSP" = trisodium orthophosphate dodecahydrate, 0.25 M sodium hydroxide. | | | |

**Table 5: Generic HDL detergent formulations: DW-BV and DW-CF**

| **Ingredient** | **Formulation** | |
|---|---|---|
| | **DW-BV** | **DW-CF** |
| Water, DI | 44.432 parts | 36.252 parts |
| Borax | 1.6 | 1.6 |
| Boric acid | 1.0 | 1.0 |
| Phosphoric acid, 75% | 0.316 | 0.316 |
| TSP¹ | 1.412 | 1.412 |
| Citric acid | 0.08 | 0.16 |
| Sodium hydroxide, 50% | 0.1 | 0.2 |
| Calcium chloride dihydrate | 0.06 | 0.06 |
| Propylene glycol | 10.0 | 10.0 |
| Ethanol, 70% | 7.0 | 7.0 |
| Hetoxol LA7 | 6.72 | 9.0 |
| Hetoxol LA4 | 1.28 | 1.7 |
| Nacconol 90G | 10.0 | 13.3 |
| Steol CS370 | 6.0 | 8.0 |
| | | |
| Total | 90.0 parts | 90.0 parts |

| | | |
|---|---|---|
| ¹ "TSP" = trisodium orthophosphate dodecahydrate, 0.25 M sodium hydroxide. | | |

The generic HDL formulations recipes disclosed in the tables above are not intended to be limiting. In one embodiment, any of them may be used as a basis for preparing commercial HDL formulations that include a variety of additional adjunct materials. However, the inhibitor-stabilized metalloprotease compositions of the present invention may be incorporated into any other suitable HDL formulations known in the art. Such HDL formulations can include a range of different combinations of buffers, surfactants, and/or other adjunct materials.

The detergent formulations of the present invention require an effective amount of metalloprotease enzyme. In some embodiments, the required level of enzyme is achieved by the addition of one or more species of metalloprotease. Typically, the detergent formulations of the present invention should comprise metalloprotease enzyme in an amount of about 0.0001-10% by weight, more preferably about 0.001-5% by weight, and most preferably about 0.01-2.0% by weight of the pre-wash (*i.e*., storage form) detergent formulation based on an enzyme that is 100% active. The activity of the enzyme must be considered when preparing any of the formulations consistent with the present invention.

In some preferred embodiments, the detergent formulations and cleaning compositions provided herein are typically formulated such that, during use in aqueous cleaning operations, the wash water has a pH of from about 5.0 to about 11.5, or in alternative embodiments, even from about 6.0 to about 10.5. In some preferred embodiments, liquid product formulations are typically formulated to have a neat pH from about 3.0 to about 9.0, while in some alternative embodiments the formulation has a neat pH from about 3 to about 5. In some embodiments, granular laundry products are typically formulated to have a pH from about 8 to about 11. Techniques for controlling pH at recommended usage levels include the use of buffers, alkalis, acids, etc., and are well known to those skilled in the art.

### Encapsulated Particle Formulations

An inhibitor-stabilized neutral metalloprotease may be employed in a granular composition or liquid, wherein the neutral metalloprotease complexed with inhibitor is in the form of an encapsulated particle to protect it from other components of the composition during storage. Encapsulation provides an additional means of controlling the availability of the inhibitor-stabilized neutral metalloprotease during the cleaning process and may enhance performance of the inhibitor-stabilized neutral metalloprotease. An encapsulated inhibitor-stabilized neutral metalloprotease of the present invention may find use in various settings.

The inhibitor-stabilized neutral metalloprotease be encapsulated using any suitable encapsulating material(s) and method(s) known in the art. The encapsulating material typically encapsulates at least part of the inhibitor-stabilized neutral metalloprotease. Typically, the encapsulating material is water-soluble and/or water-dispersible. Additionally embodiments, the encapsulating materiarial may have a glass transition temperature (Tg) of 0°C or higher. (*See e.g.,* WO 97/11151, particularly from page 6,line 25 to page 7, line 2, for more information regarding glass transition temperatures.)

The encapsulating material may be selected from the group consisting of carbohydrates, natural or synthetic gums, chitin and chitosan, cellulose and cellulose derivatives, silicates, phosphates, borates, polyvinyl alcohol, polyethylene glycol, paraffin waxes and combinations thereof. When the encapsulating material is a carbohydrate, it may be selected from the group consisting of monosaccharides, oligosaccharides, polysaccharides, and combinations thereof. The encapsulating material may be a starch. (*See e.g*., EP 0 922 499; US 4,977,252. US 5,354,559, and US 5,935,826, for descriptions of some exemplary suitable starches.)

In additional embodiments, the encapsulating material comprises a microsphere made from plastic (*e.g*., thermoplastics, acrylonitnile, methacrylonitrile, polyacrylonitrile, polymethacrylonitrile and mixtures thereof; commercially available microspheres that find use include, but are not limited to EXPANCEL® [Casco Products, Stockholm, Sweden], PM 6545, PM 6550, PM 7220, PM 7228, EXTENDOSPHERES®, and Q-CEL® [PQ Corp., Valley Forge, PA], LUXSIL® and SPHERICELI® [Potters Industries, Inc., Carlstadt, NJ and Valley Forge, PA]).

### Methods for Making and Using Detergent Formulations and Cleaning Compositions

Inhibitor-stabilized metalloprotease compositions may be formulated into any suitable detergent formulation or cleaning composition and using any suitable process chosen by the formulator. Such formulation processes are well-known in the art. *See e.g.,* U.S. 5,879,584, U.S. 5,691,297, U.S. 5,574,005, U.S. 5,569,645, U.S. 5,565,422, U.S. 5,516,448, U.S. 5,489,392, U.S. 5,486,303, U.S. 4,515,705, U.S. 4,537,706, U.S. 4,515,707, U.S. 4,550,862, U.S. 4,561,998, U.S. 4,597,898, U.S. 4,968,451, U.S. 5,565,145, U.S. 5,929,022, U.S. 6,294,514, and U.S. 6,376,445.

In preferred embodiments, the detergent formulations and cleaning compositions of the present invention find use in washing fabrics and/or surfaces. In some embodiments, at least a portion of the surface and/or fabric is contacted with at least one embodiment of the cleaning compositions of the present invention, in neat form or diluted in a wash liquor, and then the surface and/or fabric is optionally washed and/or rinsed. For purposes of the present invention, "washing" includes, but is not limited to, scrubbing, and mechanical agitation. The fabrics that may be cleaned by the formulations of the present invention comprise any fabric capable of being laundered in normal consumer use conditions.

In preferred embodiments, the detergent formulations and cleaning compositions of the present invention are used at concentrations of from about 500 ppm to about 15,000 ppm in solution. In some embodiments in which the wash solvent is water, the water temperature typically ranges from about 5 °C to about 90 °C. In some preferred embodiments for fabric cleaning, the water to fabric mass ratio is typically from about 1:1 to about 30:1.

### Adjunct Materials Useful with the Present Invention

While not essential for the purposes of the present invention, in some embodiments, the non-limiting list of adjuncts described herein are suitable for use in the cleaning compositions of the present invention. Indeed, in some embodiments, adjuncts are incorporated into the cleaning compositions of the present invention. In some embodiments, adjunct materials assist and/or enhance cleaning performance, treat the substrate to be cleaned, and/or modify the aesthetics of the cleaning composition (*e.g*., perfumes, colorants, dyes, etc.). It is understood that such adjuncts are added to the formulations and compositions comprising the inhibitor-stabilized metalloprotease compositions of the present invention. The precise nature of these additional components, and levels of incorporation thereof, depends on the physical form of the composition and the nature of the cleaning operation for which it is to be used.

Suitable adjunct materials include, but are not limited to, surfactants, builders, chelating agents, dye transfer inhibiting agents, deposition aids, dispersants, additional enzymes, and enzyme stabilizers, catalytic materials, bleach activators, bleach boosters, hydrogen peroxide, sources of hydrogen peroxide, preformed peracids, polymeric dispersing agents, clay soil removal/anti-redeposition agents, brighteners, suds suppressors, dyes, perfumes, structure elasticizing agents, fabric softeners, carriers, hydrotropes, processing aids and/or pigments.

In addition to those provided explicitly herein, additional adjunct materials are known in the art. (*See e.g.,* U.S. Patent Nos. 5,576,282, 6,306,812 B and 6,326,348 B1.) In some embodiments, the aforementioned adjunct ingredients constitute the balance of the formulations and compositions of the present invention.

Surfactants - In some embodiments, the cleaning compositions of the present invention comprise at least one surfactant or surfactant system, wherein the surfactant is selected from nonionic surfactants, anionic surfactants, cationic surfactants, ampholytic surfactants, zwitterionic surfactants, semi-polar nonionic surfactants, and mixtures thereof. Exemplary surfactants useful in the inhibitor-stabilized metalloprotease detergent formulations of the present invention, alone or in mixtures, include: C12 ethoxylates (Alfonic 1012-6, Hetoxol LA7, Hetoxol LA4), sodium alkyl benzene sulfonates (*e.g*., Nacconol 90G), and sodium laureth sulfate (*e.g*., Steol CS-370).

In some low pH cleaning composition embodiments (*e.g*., compositions having a neat pH of from about 3 to about 5), the composition typically does not contain alkyl ethoxylated sulfate, as it is believed that this type of surfactant may be hydrolyzed by the composition under such acidic conditions.

In some embodiments, the surfactant is present at a level of from about 0.1% to about 75%, while in alternative embodiments the level is from about 1% to about 50%, while in still further embodiments the level is from about 5% to about 40%, by weight of the cleaning composition.

Builders - In some embodiments, the cleaning compositions of the present invention comprise one or more detergent builders or builder systems. In some embodiments incorporating at least one builder, the cleaning compositions comprise at least about 1%, from about 3% to about 60% or even from about 5% to about 40% builder by weight of the cleaning composition.

Builders include, but are not limited to, the alkali metal, ammonium and alkanolammonium salts of polyphosphates, alkali metal silicates, alkaline earth and alkali metal carbonates, aluminosilicate builders polycarboxylate compounds, ether hydroxypolycarboxylates, copolymers of maleic anhydride with ethylene or vinyl methyl ether, 1, 3, 5-trihydroxy benzene-2, 4, 6-trisulphonic acid, and carboxymethyloxysuccinic acid, the various alkali metal, ammonium and substituted ammonium salts of polyacetic acids such as ethylenediamine tetraacetic acid and nitrilotriacetic acid, as well as polycarboxylates such as mellitic acid, succinic acid, citric acid, oxydisuccinic acid, polymaleic acid, benzene 1,3,5-tricarboxylic acid, carboxymethyloxysuccinic acid, and soluble salts thereof. Indeed, it is contemplated that any suitable builder will find use in various embodiments of the present invention.

Chelating Agents - In some embodiments, the cleaning compositions of the present invention contain at least one chelating agent, Suitable chelating agents include, but are not limited to copper, iron and/or manganese chelating agents and mixtures thereof. In embodiments in which at least one chelating agent is used, the cleaning compositions of the present invention comprise from about 0.1% to about 15% or even from about 3.0% to about 10% chelating agent by weight of the subject cleaning composition.

Deposition Aid - In some embodiments, the cleaning compositions of the present invention include at least one deposition aid. Suitable deposition aids include, but are not limited to polyethylene glycol, polypropylene glycol, polycarboxylate, soil release polymers such as polytelephthalic acid, clays such as kaolinite, montmorillonite, atapulgite, illite, bentonite, halloysite, and mixtures thereof.

Dye Transfer Inhibiting Agents - In some embodiments, the cleaning compositions of the present invention include one or more dye transfer inhibiting agents. Suitable polymeric dye transfer inhibiting agents include, but are not limited to, polyvinylpyrrolidone polymers, polyamine N-oxide polymers, copolymers of N-vinylpyitolidone and N-vinylimidazole, polyvinyloxazolidones and polyvinylimidazoles or mixtures thereof.

In embodiments in which at least one dye transfer inhibiting agent is used, the cleaning compositions of the present invention comprise from about 0.0001% to about 10%, from about 0.01% to about 5%, or even from about 0.1% to about 3% dye transfer inhibiting agent by weight of the cleaning composition.

Dispersants - In some embodiments, the cleaning compositions of the present invention contains at least one dispersants. Suitable water-soluble organic dispersant materials include, but are not limited to homo- or co-polymeric acids or their salts, in which the polycarboxylic acid comprises at least two carboxyl radicals separated from each other by not more than two carbon atoms.

Enzymes - In some embodiments, the cleaning compositions of the present invention comprise one or more detergent enzymes in addition to a metalloprotease as described herein which provide cleaning performance and/or fabric care benefits. Examples of suitable enzymes include, but are not limited to, hemicellulases, peroxidases, proteases, cellulases, xylanases, lipases, phospholipases, esterases, cutinases, pectinases, keratinases, reductases, oxidases, phenoloxidases, lipoxygenases, ligninases, pullulanases, tannases, pentosanases, malanases, ß-glucanases, arabinosidases, hyaluronidase, chondroitinase, laccase, and amylases, or mixtures thereof. In some embodiments, a combination of enzymes (*i.e.,* a "cocktail") comprising conventional applicable enzymes like protease, lipase, cutinase and/or cellulase in conjunction with amylase is used.

Enzyme Stabilizers - In some embodiments of the present invention, the enzymes used in the detergent formulations of the present invention are stabilized. It is contemplated that various techniques for enzyme stabilization will find use in the present invention. For example, in some embodiments, the enzymes employed herein are stabilized by the presence of water-soluble sources of zinc (II), calcium (II) and/or magnesium (II) ions in the finished compositions that provide such ions to the enzymes, as well as other metal ions (e.g., barium (II), scandium (II), iron (II), manganese (II), aluminum (III), Tin (II), cobalt (II), copper (II), Nickel (II), and oxovanadium (IV)).

Catalytic Metal Complexes -- In some embodiments, the cleaning compositions of the present invention contain one or more catalytic metal complexes. In some embodiments, a metal-containing bleach catalyst is used. In some preferred embodiments, the metal bleach catalyst comprises a catalyst system comprising a transition metal cation of defined bleach catalytic activity, (*e.g*., copper, iron, titanium, ruthenium, tungsten, molybdenum, or manganese cations), an auxiliary metal cation having little or no bleach catalytic activity (*e.g*., zinc or aluminum cations), and a sequestrate having defined stability constants for the catalytic and auxiliary metal cations, particularly ethylenediaminetetraacetic acid, ethylenediaminetetra (methylenephosphonic acid) and water-soluble salts thereof. (*See e.g.,* U.S. 4,430,243.)

In some embodiments, the cleaning compositions of the present invention are catalyzed by means of a manganese compound. Such compounds and levels of use are well known in the art. *(See e.g.,* U.S. 5,576,282.) In additional embodiments, cobalt bleach catalysts are used in the cleaning compositions of the present invention. Various cobalt bleach catalysts are known in the art. *(See e.g.,* U.S. 5,597,936, and U.S. 5,595,967.) Such cobalt catalysts are readily prepared by known procedures. *(See e.g.,* U.S. 5,597,936, and U.S. 5,595,967.)

In additional embodiments, the cleaning compositions of the present invention include a transition metal complex of a macropolycyclic rigid ligand ("MRL"). As a practical matter, and not by way of limitation, in some embodiments, the compositions and cleaning processes provided by the present invention are adjusted to provide on the order of at least one part per hundred million of the active MRL species in the aqueous washing medium, and in some preferred embodiments, provide from about 0.005 ppm to about 25 ppm, more preferably from about 0.05 ppm to about 10 ppm, and most preferably from about 0.1 ppm to about 5 ppm, of the MRL in the wash liquor.

Preferred transition-metals in the instant transition-metal bleach catalyst include, but are not limited to manganese, iron and chromium. Preferred MRLs also include, but are not limited to special ultra-rigid ligands that are cross-bridged (*e.g*., 5,12-diethyl-1,5,8,12-tetraazabicyclo[6.6.2]hexadecane). Suitable transition metal MRLs are readily prepared by known procedures. (*See e.g.,* WO 00/32601, and U.S. 6,225,464.)

### EXAMPLES

The following examples are provided in order to demonstrate and further illustrate certain preferred embodiments and aspects of the present invention and are not to be construed as limiting the scope thereof.

In the experimental disclosure which follows, the following abbreviations apply: °C (degrees Centigrade); rpm or RPM (revolutions per minute); Da (Dalton), kDa (kiloDaltons); g (grams); µg and ug (micrograms); mg (milligrams); ng (nanograms); µl and ul (microliters); ml (milliliters); mm (millimeters); nm (nanometers); µm and um (micrometer); M (molar); mM (millimolar); µM and uM (micromolar); U (units); MW (molecular weight); sec (seconds); min (minute); hr (hour); OD₂₈₀ (optical density at 280 nm); OD₄₀₅ (optical density at 405 nm); OD₆₀₀ (optical density at 600 nm); PAGE (polyacrylamide gel electrophoresis); EtOH (ethanol); PBS (phosphate buffered saline [150 mM NaCl, 10 mM sodium phosphate buffer, pH 7.2]); SDS (sodium dodecyl sulfate); Tris (tris(hydroxymethyl)aminomethane); TAED (N,N,N'N'-tetraacetylethylenediamine); MES (2-morpholinoethanesulfonic acid, monohydrate; f.w. 195.24; Sigma # M-3671); CaCl₂ (calcium chloride, anhydrous; f.w. 110.99; Sigma # C-4901); DMF (N,N-dimethylformamide, f.w. 73.09, d = 0.95); w/v (weight to volume); v/v (volume to volume); NprE (neutral metalloprotease); PMN (purified MULTIFECT^{®} metalloprotease).

The following assays were used in the Examples described below.

### A. Bradford assay using 96-well microtiter plates (MTPs) for NprE concentration determination.

A Bradford assay was developed in a 96-well MTP format and used to determine NprE protease concentrations for the samples used in the following examples.

In this Bradford assay system, the following chemical and reagent solutions used were: Quick Start Bradford dye reagent (BIO-RAD, #500-0205); Dilution buffer: 10mM NaCl, 0.1mM CaCl₂, 0.005% TWEEN^{®}-80.

The equipment used was a Biomek FX Robot (Beckman) and a SpectraMAX (type 340) MTP reader; the MTPs were from Costar (type 9017).

In the test, 200 µl Bradford Dye Reagent was pipetted into each well, followed by 15 µl dilution buffer. Finally 10 µl of filtered culture broth were added to the wells.

After thorough mixing, the MTPs were incubated for at least 10 minutes at room temperature. Possible air bubbles were blown away and the ODs of the wells were read at 595 nm.

To determine the protein concentration, the background reading (*i.e*., from control wells) was subtracted from the sample readings. The obtained OD₅₉₅ values provided a relative measure of the protein content in the samples. The linearity of the NprE calibration lines between 0 to 5 µg enabled the use of OD₅₉₅ nm values as a relative measure for the protein content. As the expected content of NprE in supernatant was 200-300 µg/ml, the 10µl sample volume used in the test contained less than 5 µg protein, providing values in the linear range.

### B. AGLA assay for determining NprE activity and inhibition kinetics

The "AGLA" assay described below yields reproducible neutral metalloprotease activity (*e.g*., NprE). While the assay can be adapted to a given laboratory condition, any data obtained through a modified procedure should be reconciled with results produced by the original method.

Neutral metalloproteases cleave the peptide bond between glycine and leucine of Abz-AGLA-Nba (2-aminobenzoyl-L-alanylglycyl-L-leucyl-L-alanino-4-nitrobenzylamide; f.w. 583.65; available as # H-6675 from BaChem AG, Bubendorf, Switzerland, or as catalog # 100040-598 from VWR). Free 2-aminobenzoyl-L-alanylglycine (Abz-AG) in solution has a fluorescence emission maximum at 415 nm with an excitation maximum of 340 nm. Fluorescence of Abz-AG is quenched by nitrobenzylamide in the intact Abz-AGLA-Nba molecule.

In these experiments, the liberation of Abz-AG by protease cleavage of Abz-AGLA-Nba was monitored by fluorescence spectroscopy (λ_{exc}. = 340 nm / λₑₘᵢₛ. = 415 nm). The rate of appearance of Abz-AG was a measure of proteolytic activity. Assays were performed under non-substrate limited initial rate conditions.

### Assay Equipment

A microplate mixer with temperature control (*e.g*., Eppendorf Thermomixer) was required for reproducible assay results. The assay solutions were incubated to desired temperature (*e.g*., 25°C) in the microplate mixer prior to enzyme addition. Enzyme solutions were added to the plate in the mixer, mixed vigorously and rapidly transferred to the plate reader.

A spectrofluorimeter with capability of continuous data recording, linear regression analysis, and with temperature control was used, *e.g.,* SpectraMax M5, Gemini EM (Molecular Devices, Sunnyvale, CA). The reader was always maintained at the desired temperature (*e.g*., 25°C). The reader was set for top-read fluorescence detection and the excitation was set to 350 nm and emission to 415 nm without the use of a cut-off filter. The PMT was set to medium sensitivity and 5 readings per well. Autocalibration was turned on, but only to calibrate before the first reading. The assay was measured for 3 minutes with the reading interval minimized according to the number of wells selected to be monitored. The reader was set to calculate the rate of milli-RFU/min (thousandths of relative fluorescence units per minute). The number of readings used to calculate the rate (Vₘₐₓ points) was set to the number equivalent to 2 minutes, as determined by the reading interval (*e.g.,* a reading every 10 seconds would use 12 points to calculate the rate). The max RFU was set to 50,000.

All pipetting of enzyme and substrate stock solutions was done with positive displacement pipets (Rainin Microman). Buffer, assay, and enzyme working solutions were pipetted by single or multi-channel air-displacement pipets (Rainin LTS) from tubes, reagent reservoirs or stock microplates. A repeater pipet (Eppendorf) may be used for transferring the assay solution to microplate wells when few wells are used, to minimize reagent loss. Automated pipetting instruments such as the Beckman FX or Cybio Cybi-well may also be used for transferring enzyme solutions from a working stock microplate to the assay microplate in order to initiate an entire microplate at once.

### Reagents and Solutions

Stock MES buffer - 52.6 mM MES/NaOH, 2.6 mM CaCl₂, pH 6.5: MES acid (10.28 g) and 292 mg anhydrous CaCl₂ were dissolved in approximately 900mL purified water. The solution was titrated with NaOH to pH 6.5 (at 25°C or with temperature adjustment pH probe). The pH-adjusted buffer was made up to 1L total volume. The final solution was filtered through a 0.22 µm sterile filter and kept at room temperature.

Enzyme dilution buffer - 50 mM MES, 2.5 mM CaCl₂, pH 6.5: This buffer was produced by adding 5 mL purified water to 95 mL stock MES buffer.

Enzyme stock solution: purified NprE enzyme was diluted with enzyme dilution buffer to a concentration of approximately 1 ppm (1 µg/mL). MULTIFECT^{®} neutral metalloprotease (wild-type NprE) was diluted to concentrations below 6 ppm (6 µg/mL). Serial dilutions were preferred. Solutions were stable at room temperature for 1 hour, but for longer term storage, the solutions were maintained on ice.

Substrate stock solution - 48 mM Abz-AGLA-Nba in DMF: Approximately 28 mg of Abz-AGLA-Nba was placed in a small tube. It was dissolved in approximately 1 mL of DMF (volume will vary depending upon Abz-AGLA-Nba massed) and vortexed for several minutes. The solution was stored at room temperature shielded from light. Abz-AGLA-Nba was dissolved in DMF and was used the same day it was prepared.

Substrate dilution buffer - 50 mM MES, 2.5 mM CaCl₂, 5% DMF, pH 6.5: Five mL pure DMF were added to 95 mL stock MES buffer. This buffer was used to determine kinetic parameters.

Assay solution - 50 mM MES, 2.5 mM CaCl₂, 5% DMF, 2.4 mM Abz-AGLA-Nba pH 6.5: One mL of the substrate stock solution was added to 19 mL substrate dilution buffer and vortexed. The solution was stored at room temperature shielded from light.

### Assay Procedure

All buffers, stock, and working solutions were prepared. Each enzyme dilution was assayed in triplicate, unless otherwise indicated. When not completely full, the enzyme working solution stock microplate was arranged in full vertical columns starting from the left of the plate (to accommodate the plate reader). The corresponding assay plate was similarly set up. The microplate spectrofluorimeter was set up as previously described.

First, 200 µL aliquots of assay solution were placed in the wells of a 96-well microplate. The plate was incubated for 10 min at 25°C in a temperature controlled microplate mixer, shielded from light. The assay was initiated by transferring 10 µL of the working enzyme solutions from the stock microplate to the assay microplate in the mixer. Optimally, 96-well pipetting was used, or an 8-well multi-channel pipet was used to transfer from the left-most column first. The solutions were vigorously mixed for 15 seconds (900 rpm in Eppendorf Thermomixer). Immediately, the assay microplate was transferred to the microplate spectrofluorimeter and recording of fluorescence measurements at excitation of 350 nm and emission of 415 nm were begun. The spectrofluorimeter software calculated the reaction rates of the increase in fluorescence for each well to a linearly regressed line of milli-RFU/min. In some experiments, a second plate was placed in the microplate mixer for temperature equilibration while the first plate was being read.

The initial velocities were linear with respect to product concentration (*i.e*., liberated 2-aminobenzoyl fluorescence) up to 0.3 mM product, which corresponded to approximately 50,000 RFU in a solution starting at 2.3 mM Abz-AGLA-Nba with background fluorescence of approximately 22,000 RFU.

### EXAMPLE 1

### Increased NprE stability with higher storage concentration

This example illustrates the increase in neutral metalloprotease stability that occurs when the enzyme is maintained at higher concentrations, and/or in the presence of 10% propylene glycol (PPG) and CaCl₂.

Samples were prepared containing the neutral metalloprotease, NprE, at a range of concentrations from 625 to 10000 ppm in 10 mM HEPES (N-(2-Hydroxyethyl)piperazine-N'-(2-ethanesulfonic acid)) buffer at pH 8.0. All samples, except the one control at 625 ppm NprE concentration, included 10% PPG and 0.5 mM CaCl₂. All samples were incubated at a temperature of 32 °C over a period of 6 hours. NprE activity of the samples was measured using the AGLA activity assay at various time points.

### Results

As shown in Figure 1, the control sample with 625 ppm NprE and no added PPG or CaCl₂ lost nearly all of its activity within the first 2 hours. In contrast the NprE activity of the other samples maintained most of their activity in correlation with higher protein concentration. At 10,000 µg/mL (or ppm) NprE concentration, the enzyme maintains almost full activity for 1.5 hours before losing activity. In comparison, 625, 1250 and 2500 ppm protein sample showed decreased activity (~60% at 1.5 hr). Compared to the control sample, the high concentration sample showed marked increase in the storage stability.

These results showing correlation of increased NprE stability with increased concentration are consistent with stabilization by product inhibition.

### EXAMPLE 2

### NprE inhibition by casein hydrolysis product

This example illustrates the use of a neutral metalloprotease to generate protein hydrolysis products which are then used to stabilize the neutral metalloprotease via competitive inhibition.

### Materials and Methods

Casein from bovine milk casein (catalog #C 7078; Sigma Chemical, St. Louis, MO) at 100 mg/mL was incubated with 0.4 mg/mL of the neutral metalloprotease, NprE in 10 mL of buffer (50mM MES, 2.5 mM CaCl₂, pH 6.5) overnight in a shaking incubated set at 32°C. The resulting digestion mix was centrifuged at 18,000 rpm with Sorvall RC-5B Plus centrifuge (Thermo Fisher Scientific, Inc., Waltham MA) equipped with SM-24 fixed angle rotor at 4°C for 30 minutes to remove any un-reacted particulates. After centrifugation, the supernatant was filtered through a 5K MWCO membrane using a Vivaspin 20 centrifugal filter device (Sartorius AG, Germany). The flow through material, which should include only the hydrolysis products (also referred to herein as "casein peptide") with molecular weight less than about 5000 Da, was collected.

Quantification of the casein hydrolysis products was carried out using trinitrobenzene sulfonic acid (TNBS) assay, which colorimetrically determines free amines present in the peptides of the mixture using free amino acid as a standard. Typically, 10 µL samples were mixed with 60 µL of 1.2 mg/mL TNBS in 120 mM borate buffer, pH 9 and incubated for 15 minutes at 50 °C. The reaction mixtures were then neutralized with 140 µL of 500 mM phosphate buffer, pH 7.5. The color changes were monitored at 420 nm, calibrated against amino acid standards (catalog #AAS18; Sigma Chemical, St. Louis, MO). The quantified casein hydrolysis product mixture was used to generate a stock solution used to carry out the NprE inhibition kinetics study below.

The kinetics of NprE inhibition by the casein hydrolysis product mixture was carried out using the general AGLA activity assay described above but with varying concentrations of the casein hydrolysis product present in the assay mix. Standard Michaelis-Menten enzymatic rate plots were prepared and used to derive the various kinetic constants including the apparent Kᵢ for the casein hydrolysis product. The assay solution was 50 mM MES buffer with 2.5 mM CaCl₂ and 0.005% Tween 80 at pH 6.5 at room temperature.

### Results

Figure 2 shows inhibition of NprE by the casein hydrolysis products generated and isolated as described above. Figures 2A-2D shows the standard Michaelis-Menten kinetic plots for casein hydrolysis product inhibition against the fluorogenic AGLA substrate. Figure 2B shows that the double reciprocal plots share a common y-intercept indicating that the hydrolysis product acts as a competitive inhibitor of NprE. Figures 2C and 2D depict the apparent Kₘ and double reciprocal plot slope replots, respectively.

These results demonstrate that the casein hydrolysis product mixture generated by digestion of milk casein by NprE also is a protein hydrolysate inhibitor of NprE with an apparent K*i* of - 10 mM.

### EXAMPLE 3

### Increased NprE stability in liquid detergent formulations

This example illustrates the use of a range of protein hydrolysates to stabilize a neutral metalloprotease containing detergent formulation.

### Materials

The following protein hydrolysates were obtained from Sigma Chemical (St. Louis, MO) and used without further purification: HyPep 4601™ protein hydrolysate from wheat gluten (catalog #H 6784), Amisoy, soy protein acid hydrolysate (catalog # S 1674), Amicase, bovine milk casein acid hydrolysate (catalog # A 2427), and Proteose Peptone, enzymatic hydrolysate from vegetable protein (catalog # P 0431). Casein from bovine milk (catalog # C7078; Sigma Chemical, St. Louis, MO) was used for hydrolysis by NprE.

### Protein hydrolysate stock solutions

Protein hydrolysate stock solutions were prepared using the commercially obtained reagents at 70 mg/ml concentration in 10 mM HEPES buffer at pH 8.0.

Bovine milk casein hydrolysate was generated by digesting casein with 8 mg/mL NprE in 50mM MES buffer with 2.5 mM CaCl₂, pH 6.5, at 37°C. Undigested material was removed by centrifugation followed by dialysis with MWCO 5 kDa membrane. Flow through material was collected, aliquoted and stored at -20°C for further use. Casein hydrolysate product was determined to be -90 mM peptide by trinitrobenzene sulfonic acid (TNBS) peptide assay (as in Example 2).

### Heavy Duty Liquid (HDL) detergent preparation

The HDL detergent formulation used in this example was: DW-CT. This formulation has a 37% water content and was designed to make up to 90% by volume with 10% room (by volume) to add ingredients such as stabilizers or enzymes. It was prepared according to the recipe shown in Table 1 above.

Assays were carried out using a 10% DW-CT detergent formulation made by mixing 5 mL of DW-CT (prepared as in Table 1), 1 mL of 500 mM HEPES at pH 8.0, and 44 mL distilled water.

### Stability assay sample preparation

Stability assay samples were prepared using five different candidate protein hydrolysates, as well as a control with no inhibitor added.

In general, 20 µL of inhibitor stock solution was pre-mixed with 10 µL of 50 mg/mL NprE stock solution. Then, 220 µL of 10% DW-CT detergent formulation in 10mM HEPES was added to each sample so that the final NprE concentration was 2 mg/mL. Samples were incubated at 32°C in a microtiter plate in a Thermomixer (Eppendorf).

The final concentration of enzyme in each sample was 2 mg/mL NprE. The final inhibitor concentrations in their respective samples were: 5.6 mg/mL of Amisoy, Amicase, HyPep 4601, or Proteose Peptone, respectively; or 7.2 mM of the casein hydrolysis product mixture (based on 12.5-fold dilution). The final dilution of the DW-CT detergent formulation was 9%.

### Remaining AGLA activity assay

Remaining NprE activity of each of stability assay samples (prepared as above) was measured at various time points using the general AGLA activity assay except that the assay solution used was 50 mM MES, 2.5 mM CaCl₂, 0.005% Tween 80 at pH 6.5. It was found that the remaining AGLA activity assay was linear over a 9000-fold dilution range.

### SDS-PAGE of stability assay samples

As an independent measure of the level of protection against NprE autolysis afforded by the inhibitors, SDS-PAGE analysis was carried out for each stability assay sample to determine the relative amount of intact NprE remaining versus autolysis products.

At the end of stability assay incubation period (t = 200 min), 10 µL of each sample was taken out and quenched with 200 µL 1N HCl. Immediately, 200 µL of 5% TCA was added. TCA precipitation was carried out on ice for 20 minutes. The pellet was collected by centrifugation and further washed with ice cold 90% acetone. The pellet was then resuspended in 1.5X sample loading buffer, and heated at 95°C for 5 minutes. The samples were then loaded on a 4-12% SDS-PAGE gel. Electrophoresis and gel visualization was carried out with Coomassie blue stain were carried out using SDS-PAGE standard protocols well known in the art.

### Results

As shown in Figure 3, the metalloprotease NprE loses more than 80% of its original activity in about an hour when incubated in the HDL detergent formulation, DW-CT. The addition of protein hydrolysate inhibitors to the same NprE detergent formulation significantly improved the enzyme's stability. For example, the detergent formulations containing Amisoy (soy protein acid hydrolysate), NprE digested casein hydrolysate, HyPep 4601™ (wheat gluten hydrolysate), Amicase (casein acid hydrolysate), and Proteose peptone (vegetable protein hydrolysate) all showed significant remaining NprE activity. Amisoy resulted in the most stable NprE HDL detergent formulation with over 50% remaining activity after 3 hours. The formulation with the added casein hydrolysis product also performed very well, still exhibiting 40% activity after 3 hours.

The SDS-PAGE results of the stability assay samples at 200 minutes indicated that protein hydrolysates Amisoy and casein hydrolysis product provided the strongest protection against NprE autolysis in the detergent formulation. These protein hydrolysates' ability to stabilize was evidenced by the fact that the SDS-PAGE for these samples showed a single strong NprE band with no detectable low molecular weight bands indicating autolysis products. Indeed, the intensity of the NprE band was comparable to that control NprE sample that was not incubated. In comparison, the SDS-PAGE of that NprE sample incubated without any inhibitor showed almost no detectable NprE band, indicating that the unprotected NprE was almost completely degraded after 200 minutes in the detergent formulation. The HyPep 4601 protein hydrolysate sample also showed a single strong NprE band with little or no visible low molecular weight products, although the band was not as strong as for Amisoy and casein hydrolysis products. The protein hydrolysates Amicase and Proteose peptone showed NprE bands in SDS-PAGE, but only slightly brighter than the NprE band in the sample incubated without inhibitors. Thus, the SDS-PAGE results are consistent with the remaining NprE activity as measured using the AGLA assay (shown in Figure 3).

All patents and publications mentioned in the specification are indicative of the levels of those skilled in the art to which the invention pertains.

While particular embodiments of the present invention have been illustrated and described, it will be apparent to those skilled in the art that various other changes and modifications can be made within the scope of the claims.

The invention illustratively described herein suitably may be practiced in the absence of any element or elements, limitation or limitations which is not specifically disclosed herein. The terms and expressions which have been employed are used as terms of description and not of limitation, and there is no intention that in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the concepts herein disclosed may be resorted to by those skilled in the art, and that such modifications and variations are considered to be within the scope of this invention as defined by the appended claims.

## Claims

1. A liquid detergent formulation comprising:
(a) from about 1% to about 75% of a surfactant by weight;
(b) from about 10% to about 95% of water by weight;
(c) from about 0.01% to about 5% of a neutral metalloprotease by weight; and
(d) an amount of neutral metalloprotease inhibitor such that the inhibitor binds to at least about 90% of the neutral metalloprotease molecules prior to use, and wherein appropriate dilution of the detergent formulation results in the inhibitor dissociating from at least about 25%, preferably at least about 45%, of the bound neutral metalloprotease molecules, wherein said neutral metalloprotease inhibitor is a protein hydrolysate.

2. The formulation of claim 1 wherein said protein hydrolysate is selected from wheat gluten hydrolysate, soy protein acid hydrolysate, casein acid hydrolysate from bovine milk, enzymic hydrolysate from vegetable protein, and any combination thereof.

3. The formulation of claim 1, wherein the amount of inhibitor is between about 0.01% and about 15% by weight.

4. The formulation of claim 1, wherein the detergent formulation comprises a heavy duty liquid (HDL) formulation.

5. The formulation of claim 1, wherein the liquid detergent formulation further comprises about 5% to about 10% polypropylene glycol and/or further comprises about 0.5 mM to about 5 mM calcium ions.

6. The formulation of claim 1, wherein the liquid detergent formulation does not comprise boron.

7. The formulation of any one of claims 1 to 6, wherein the neutral metalloprotease is isolated from a *Bacillus sp.*

8. The formulation of any one of claims 1 to 6 wherein the neutral metalloprotease is from *B. amyloliquefaciens, B. cereus, B. cereus* E33L, *B. caldolyticus, B. pumulis, B. megaterium, B.subtilis amylosacchariticus, Brevibacillus brevis, Paenibacillus polymyxa (Bacillus polymyxa), B. stearothermophilus, B. thuringiensis, B. subtilis* or *S*. *aureus*

9. The formulation of any one of claims 1 to 6 wherein the neutral metalloprotease is NprE, aureolysin, extracellular elastase or neutal protease B.

10. The formulation of any one of the preceding claims, wherein the protein hydrolysate is generated by digestion of said protein with at least one neutral metalloprotease.

11. The formulation of claim 10 wherein the protein hydrolysate is generated by the same neutral metalloprotease as is present in the detergent formulation.

12. The formulation of any one of claims 8 to 11 wherein the hydrolysate comprises protein fragments of less than about 5000 Da.

13. The formulation of any one of claims 8 to 12 wherein the protein is casein.

14. The formulation of claim 1, wherein the formulation is an encapsulated particle.

15. A method for preparing an inhibitor-stabilized liquid detergent formulation comprising:
(a) incubating a mixture comprising at least one neutral metalloprotease and a protein substrate in an aqueous buffer at pH between about 6.5 and about 11 and temperature from about 22°C to about 37°C, whereby digestion of the substrate protein by the metalloprotease generates a hydrolysis product;
(b) isolating the hydrolysis product with molecular weight less than about 5000 Da; and
(c) combining the hydrolysis product of step (b) with a liquid detergent formulation comprising from about 0.001% to about 10% of a neutral metalloprotease by weight.

16. The method of claim 15, wherein the incubation mixture comprises from about 0.001% to about 10% neutral metalloprotease and from about 5% to about 20% protein substrate by weight.

17. The method of claim 15, wherein the hydrolysis product is generated by the same neutral metalloprotease as is present in the detergent formulation.

18. The method of claim 17, wherein the neutral metalloprotease is NprE and the protein substrate is casein.

19. The method of claim 15, wherein the detergent formulation comprises an HDL detergent formulation.

## Patentansprüche

1. Flüssige Detergensformulierung, umfassend:
(a) von etwa 1 Gew.-% bis etwa 75 Gew.-% eines Tensids;
(b) von etwa 10 Gew.-% bis etwa 95 Gew.-% Wasser;
(c) von etwa 0,01 Gew.-% bis etwa 5 Gew.-% einer neutralen Metalloprotease; und
(d) eine Menge von neutralem Metalloprotease-Inhibitor, derart, dass vor der Verwendung der Inhibitor an mindestens etwa 90% der neutralen Metalloprotease-Moleküle bindet und wobei passende Verdünnung der Detergensformulierung dazu führt, dass der Inhibitor von mindestens etwa 25%, vorzugsweise mindestens etwa 45%, der gebundenen neutralen Metalloprotease-Moleküle dissoziiert, wobei der neutrale Metalloprotease-Inhibitor ein Proteinhydrolysat ist.

2. Formulierung nach Anspruch 1, wobei das Proteinhydrolysat aus Weizengluten-Hydrolysat, Sojaprotein-Säurehydrolysat, Casein-Säurehydrolysat aus Rindermilch, enzymatischem Hydrolysat aus Pflanzenprotein und einer beliebigen Kombination davon ausgewählt ist.

3. Formulierung nach Anspruch 1, wobei die Menge von Inhibitor zwischen etwa 0,01 Gew.-% und etwa 15 Gew.-% liegt.

4. Formulierung nach Anspruch 1, wobei die Detergensformulierung eine flüssige Hochleistungs-(HDL)-Formulierung umfasst.

5. Formulierung nach Anspruch 1, wobei die flüssige Detergensformulierung weiterhin etwa 5% bis etwa 10% Polypropylenglycol umfasst und/oder weiterhin etwa 0,5 mM bis etwa 5 mM Calciumionen umfasst.

6. Formulierung nach Anspruch 1, wobei die flüssige Detergensformulierung kein Bor umfasst.

7. Formulierung nach einem der Ansprüche 1 bis 6, wobei die neutrale Metalloprotease aus einer *Bacillus sp.* isoliert wird.

8. Formulierung nach einem der Ansprüche 1 bis 6, wobei die neutrale Metalloprotease aus *B. amyloliquefaciens, B. cereus, B. cereus E33L, B. caldolyticus, B. pumulis, B. megaterium, B subtilis amylosacchariticus, Brevibacillus brevis, Paenibacillus polymyxa (Bacillus polymyxa), B. stearothermophilus, B. thuringiensis, B. subtilis* oder *S*. *aureus* stammt.

9. Formulierung nach einem der Ansprüche 1 bis 6, wobei die neutrale Metalloprotease NprE, Aureolysin, extrazelluläre Elastase oder neutrale Protease B ist.

10. Formulierung nach einem der vorhergehenden Ansprüche, wobei das Proteinhydrolysat durch Digestion des Proteins mit mindestens einer neutralen Metalloprotease generiert wird.

11. Formulierung nach Anspruch 10, wobei das Proteinhydrolysat durch die gleiche neutrale Metalloprotease generiert wird, wie sie in der Detergensformulierung vorhanden ist.

12. Formulierung nach einem der Ansprüche 8 bis 11, wobei das Hydrolysat Proteinfragmente von weniger als etwa 5000 Da umfasst.

13. Formulierung nach einem der Ansprüche 8 bis 12, wobei das Protein Casein ist.

14. Formulierung nach Anspruch 1, wobei die Formulierung ein eingekapseltes Partikel ist.

15. Verfahren zum Herstellen einer Inhibitor-stabilisierten flüssigen Detergensformulierung, umfassend:
(a) Inkubieren eines Gemisches, umfassend mindestens eine neutrale Metalloprotease und ein Proteinsubstrat in einem wässerigen Puffer bei einem pH zwischen etwa 6,5 und etwa 11 und einer Temperatur von etwa 22°C bis etwa 37°C, wodurch Digestion des Substratproteins durch die Metalloprotease ein Hydrolyseprodukt generiert;
(b) Isolieren des Hydrolyseprodukts mit einem Molekulargewicht von weniger als etwa 5000 Da; und
(c) Vereinigen des Hydrolyseprodukts von Schritt (b) mit einer flüssigen Detergensformulierung, umfassend von etwa 0,001 Gew.-% bis etwa 10 Gew.-% einer neutralen Metalloprotease.

16. Verfahren nach Anspruch 15, wobei das Inkubationsgemisch von etwa 0,001 Gew.-% bis etwa 10 Gew.-% neutrale Metalloprotease und von etwa 5 Gew.-% bis etwa 20 Gew.-% Proteinsubstrat umfasst.

17. Verfahren nach Anspruch 15, wobei das Hydrolyseprodukt durch die gleiche neutrale Metalloprotease generiert wird, wie sie in der Detergensformulierung vorhanden ist.

18. Verfahren nach Anspruch 17, wobei die neutrale Metalloprotease NprE ist und das Proteinsubstrat Casein ist.

19. Verfahren nach Anspruch 15, wobei die Detergensformulierung eine HDL-Detergensformulierung umfasst.

## Revendications

1. Formulation de détergent liquide comprenant:
(a) d'environ 1 % à environ 75% d'un tensioactif en poids;
(b) d'environ 10% à environ 95% d'eau en poids;
(c) d'environ 0,01% à environ 5% d'une métalloprotéase neutre en poids; et
(d) une quantité d'inhibiteur de métalloprotéase neutre de sorte que l'inhibiteur se lie à au moins environ 90% des molécules de métalloprotéase neutre avant usage et où une dilution appropriée de la formulation de détergent conduit à la dissociation de l'inhibiteur à partir d'au moins environ 25%, de préférence au moins environ 45%, des molécules de métalloprotéase neutre liées, où ledit inhibiteur de métalloprotéase neutre est un hydrolysat de protéine.

2. Formulation selon la revendication 1, dans laquelle ledit hydrolysat de protéine est choisi parmi un hydrolysat de gluten de blé, un hydrolysat d'acide de protéine de soja, un hydrolysat d'acide de caséine issu d'un lait bovin, un hydrolysat enzymatique issu d'une protéine végétale et toute combinaison de ceux-ci.

3. Formulation selon la revendication 1, dans laquelle la quantité d'inhibiteur est entre environ 0,01% et environ 15% en poids.

4. Formulation selon la revendication 1, où la formulation de détergent comprend une formulation de liquide ultra-résistant (HDL).

5. Formulation selon la revendication 1, où la formulation de détergent liquide comprend en outre d'environ 5% à environ 10% de polypropylèneglycol et/ou comprend en outre d'environ 0,5 mM à environ 5 mM d'ions calcium.

6. Formulation selon la revendication 1, où la formulation de détergent liquide ne comprend pas de bore.

7. Formulation selon l'une quelconque des revendications 1 à 6, dans laquelle la métalloprotéase neutre est isolée à partir d'une espèce de *Bacillus.*

8. Formulation selon l'une quelconque des revendications 1 à 6, dans laquelle la métalloprotéase neutre est issue de *B. amyloliquefaciens, B. cereus, B. cereus* E33L, *B*. *caldolyticus, B. pumulis, B. megaterium, B. substilis amylosacchariticus, Brevibacillus brevis, Paenibacillus polymyxa (Bacillus polymyxa), B. steraothermophilus, B. thuringiensis, B. subtilis* ou *S*. *aureus.*

9. Formulation selon l'une quelconque des revendications 1 à 6, dans laquelle la métalloprotéase neutre est NprE, une auréolysine, une élastase extracellulaire ou une protéase B neutre.

10. Formulation selon l'une quelconque des revendications précédentes, dans laquelle l'hydrolysat de protéine est généré par une digestion de ladite protéine avec au moins une métalloprotéase neutre.

11. Formulation selon la revendication 10, dans laquelle l'hydrolysat de protéine est généré par la même métalloprotéase neutre que celle qui est présente dans la formulation de détergent.

12. Formulation selon l'une quelconque des revendications 8 à 11, dans laquelle l'hydrolysat comprend des fragments de protéine de moins d'environ 5000 Da.

13. Formulation selon l'une quelconque des revendications 8 à 12, dans laquelle la protéine est une caséine.

14. Formulation selon la revendication 1, où la formulation est une particule encapsulée.

15. Procédé pour la préparation d'une formulation de détergent liquide stabilisée par un inhibiteur comprenant:
(a) l'incubation d'un mélange comprenant au moins une métalloprotéase neutre et un substrat de protéine dans un tampon aqueux à un pH entre environ 6,5 et environ 11 et une température d'environ 22°C à environ 37°C, en conséquence de quoi une digestion de la protéine du substrat par la métalloprotéase génère un produit d'hydrolyse;
(b) l'isolement du produit d'hydrolyse avec un poids moléculaire de moins d'environ 5000 Da; et
(c) la combinaison du produit d'hydrolyse de l'étape (b) avec une formulation de détergent liquide comprenant d'environ 0,001% à environ 10% d'une métalloprotéase neutre en poids.

16. Procédé selon la revendication 15, dans lequel le mélange d'incubation comprend d'environ 0,001% à environ 10% de métalloprotéase neutre et d'environ 5% à environ 20% de substrat de protéine en poids.

17. Procédé selon la revendication 15, dans lequel le produit d'hydrolyse est généré par la même métalloprotéase neutre que celle qui est présente dans la formulation de détergent.

18. Procédé selon la revendication 17, dans lequel la métalloprotéase neutre est NprE et le substrat de protéine est une caséine.

19. Procédé selon la revendication 15, dans lequel la formulation de détergent comprend une formulation de détergent HDL.
